# EUROPEAN PATENT APPLICATION

(11) **EP 3 722 426 A1**
(43) Date of publication of application: **14.10.2020**
(21) Application number: 18886810.3
(22) Date of filing: 05.12.2018
(51) Int. Cl.: C12N 15/67, C12N 15/85, C12N 9/10, A61K 48/00, A61K 38/00

(54) **FACTOR VIII VARIANT-EXPRESSING VECTOR WITH INCREASED PROTEIN EXPRESSION CAPACITY**

(30) Priority: 07.12.2017 KR 20170167405
(71) Applicant: G&P Bioscience Co., Ltd., Goyang-si, Gyeonggi-do 10326 (KR); Reyon Pharmaceutical Co., Ltd, Seoul 06176 (KR)
(72) Inventor: HO, Seong-Hyun, Seoul 08052 (KR); PARK, Su Jin, Gwangmyeong-si Gyeonggi-do 14256 (KR)
(74) Representative: V.O.
(86) International application number: PCT/KR2018/015333
(87) International publication number: WO 2019/112322

(57) **Abstract**

The present disclosure relates to an expression vector that carries a polynucleotide coding for a coagulation factor VIII mutant and has increased protein expression and a pharmaceutical composition containing the expression vector for preventing or treating hemorrhagic disease or hemorrhage.

The factor VIII mutant of the present disclosure is derived by deleting a part of the B-domain (residues 784-1667) and a part of the a3 region (residues 1668-1671) in factor VIII and, as a result, an expression vector carrying a polynucleotide coding for the factor VIII mutant has remarkably increased protein expression.

<110>	GNPBIOSCIENCE

<120>	Factor 8 mutant expression vector with inhanced protein
	expression

<130>	HPC-7658

<160>	14

<170>	KoPatentIn 3.0

<210>	1
<211>	2351
<212>	PRT
<213>	Homo sapiens

<400>	1
Met Gln Ile Glu Leu Ser Thr Cys Phe Phe Leu Cys Leu Leu Arg Phe
  1               5                  10                  15

Cys Phe Ser Ala Thr Arg Arg Tyr Tyr Leu Gly Ala Val Glu Leu Ser
             20                  25                  30

Trp Asp Tyr Met Gln Ser Asp Leu Gly Glu Leu Pro Val Asp Ala Arg
         35                  40                  45

Phe Pro Pro Arg Val Pro Lys Ser Phe Pro Phe Asn Thr Ser Val Val
     50                  55                  60

Tyr Lys Lys Thr Leu Phe Val Glu Phe Thr Asp His Leu Phe Asn Ile
 65                  70                  75                  80

Ala Lys Pro Arg Pro Pro Trp Met Gly Leu Leu Gly Pro Thr Ile Gln
                 85                  90                  95

Ala Glu Val Tyr Asp Thr Val Val Ile Thr Leu Lys Asn Met Ala Ser
            100                 105                 110

His Pro Val Ser Leu His Ala Val Gly Val Ser Tyr Trp Lys Ala Ser
        115                 120                 125

Glu Gly Ala Glu Tyr Asp Asp Gln Thr Ser Gln Arg Glu Lys Glu Asp
    130                 135                 140

Asp Lys Val Phe Pro Gly Gly Ser His Thr Tyr Val Trp Gln Val Leu
145                 150                 155                 160

Lys Glu Asn Gly Pro Met Ala Ser Asp Pro Leu Cys Leu Thr Tyr Ser
                165                 170                 175

Tyr Leu Ser His Val Asp Leu Val Lys Asp Leu Asn Ser Gly Leu Ile
            180                 185                 190

Gly Ala Leu Leu Val Cys Arg Glu Gly Ser Leu Ala Lys Glu Lys Thr
        195                 200                 205

Gln Thr Leu His Lys Phe Ile Leu Leu Phe Ala Val Phe Asp Glu Gly
    210                 215                 220

Lys Ser Trp His Ser Glu Thr Lys Asn Ser Leu Met Gln Asp Arg Asp
225                 230                 235                 240

Ala Ala Ser Ala Arg Ala Trp Pro Lys Met His Thr Val Asn Gly Tyr

                245                 250                 255

Val Asn Arg Ser Leu Pro Gly Leu Ile Gly Cys His Arg Lys Ser Val
            260                 265                 270

Tyr Trp His Val Ile Gly Met Gly Thr Thr Pro Glu Val His Ser Ile
        275                 280                 285

Phe Leu Glu Gly His Thr Phe Leu Val Arg Asn His Arg Gln Ala Ser
    290                 295                 300

Leu Glu Ile Ser Pro Ile Thr Phe Leu Thr Ala Gln Thr Leu Leu Met
305                 310                 315                 320

Asp Leu Gly Gln Phe Leu Leu Phe Cys His Ile Ser Ser His Gln His
                325                 330                 335

Asp Gly Met Glu Ala Tyr Val Lys Val Asp Ser Cys Pro Glu Glu Pro
            340                 345                 350

Gln Leu Arg Met Lys Asn Asn Glu Glu Ala Glu Asp Tyr Asp Asp Asp
        355                 360                 365

Leu Thr Asp Ser Glu Met Asp Val Val Arg Phe Asp Asp Asp Asn Ser
    370                 375                 380

Pro Ser Phe Ile Gln Ile Arg Ser Val Ala Lys Lys His Pro Lys Thr
385                 390                 395                 400

Trp Val His Tyr Ile Ala Ala Glu Glu Glu Asp Trp Asp Tyr Ala Pro
                405                 410                 415

Leu Val Leu Ala Pro Asp Asp Arg Ser Tyr Lys Ser Gln Tyr Leu Asn
            420                 425                 430

Asn Gly Pro Gln Arg Ile Gly Arg Lys Tyr Lys Lys Val Arg Phe Met
        435                 440                 445

Ala Tyr Thr Asp Glu Thr Phe Lys Thr Arg Glu Ala Ile Gln His Glu
    450                 455                 460

Ser Gly Ile Leu Gly Pro Leu Leu Tyr Gly Glu Val Gly Asp Thr Leu
465                 470                 475                 480

Leu Ile Ile Phe Lys Asn Gln Ala Ser Arg Pro Tyr Asn Ile Tyr Pro
                485                 490                 495

His Gly Ile Thr Asp Val Arg Pro Leu Tyr Ser Arg Arg Leu Pro Lys
            500                 505                 510

Gly Val Lys His Leu Lys Asp Phe Pro Ile Leu Pro Gly Glu Ile Phe
        515                 520                 525

Lys Tyr Lys Trp Thr Val Thr Val Glu Asp Gly Pro Thr Lys Ser Asp
    530                 535                 540

Pro Arg Cys Leu Thr Arg Tyr Tyr Ser Ser Phe Val Asn Met Glu Arg
545                 550                 555                 560

Asp Leu Ala Ser Gly Leu Ile Gly Pro Leu Leu Ile Cys Tyr Lys Glu
                565                 570                 575

Ser Val Asp Gln Arg Gly Asn Gln Ile Met Ser Asp Lys Arg Asn Val

580                 585                 590

Ile Leu Phe Ser Val Phe Asp Glu Asn Arg Ser Trp Tyr Leu Thr Glu
        595                 600                 605

Asn Ile Gln Arg Phe Leu Pro Asn Pro Ala Gly Val Gln Leu Glu Asp
    610                 615                 620

Pro Glu Phe Gln Ala Ser Asn Ile Met His Ser Ile Asn Gly Tyr Val
625                 630                 635                 640

Phe Asp Ser Leu Gln Leu Ser Val Cys Leu His Glu Val Ala Tyr Trp
                645                 650                 655

Tyr Ile Leu Ser Ile Gly Ala Gln Thr Asp Phe Leu Ser Val Phe Phe
            660                 665                 670

Ser Gly Tyr Thr Phe Lys His Lys Met Val Tyr Glu Asp Thr Leu Thr
        675                 680                 685

Leu Phe Pro Phe Ser Gly Glu Thr Val Phe Met Ser Met Glu Asn Pro
    690                 695                 700

Gly Leu Trp Ile Leu Gly Cys His Asn Ser Asp Phe Arg Asn Arg Gly
705                 710                 715                 720

Met Thr Ala Leu Leu Lys Val Ser Ser Cys Asp Lys Asn Thr Gly Asp
                725                 730                 735

Tyr Tyr Glu Asp Ser Tyr Glu Asp Ile Ser Ala Tyr Leu Leu Ser Lys
            740                 745                 750

Asn Asn Ala Ile Glu Pro Arg Ser Phe Ser Gln Asn Ser Arg His Pro
        755                 760                 765

Ser Thr Arg Gln Lys Gln Phe Asn Ala Thr Thr Ile Pro Glu Asn Asp
    770                 775                 780

Ile Glu Lys Thr Asp Pro Trp Phe Ala His Arg Thr Pro Met Pro Lys
785                 790                 795                 800

Ile Gln Asn Val Ser Ser Ser Asp Leu Leu Met Leu Leu Arg Gln Ser
                805                 810                 815

Pro Thr Pro His Gly Leu Ser Leu Ser Asp Leu Gln Glu Ala Lys Tyr
            820                 825                 830

Glu Thr Phe Ser Asp Asp Pro Ser Pro Gly Ala Ile Asp Ser Asn Asn
        835                 840                 845

Ser Leu Ser Glu Met Thr His Phe Arg Pro Gln Leu His His Ser Gly
    850                 855                 860

Asp Met Val Phe Thr Pro Glu Ser Gly Leu Gln Leu Arg Leu Asn Glu
865                 870                 875                 880

Lys Leu Gly Thr Thr Ala Ala Thr Glu Leu Lys Lys Leu Asp Phe Lys
                885                 890                 895

Val Ser Ser Thr Ser Asn Asn Leu Ile Ser Thr Ile Pro Ser Asp Asn
            900                 905                 910

Leu Ala Ala Gly Thr Asp Asn Thr Ser Ser Leu Gly Pro Pro Ser Met

915                         920                         925

Pro Val His Tyr Asp Ser Gln Leu Asp Thr Thr Leu Phe Gly Lys Lys
    930                         935                         940

Ser Ser Pro Leu Thr Glu Ser Gly Gly Pro Leu Ser Leu Ser Glu Glu
945                         950                         955                         960

Asn Asn Asp Ser Lys Leu Leu Glu Ser Gly Leu Met Asn Ser Gln Glu
                        965                         970                         975

Ser Ser Trp Gly Lys Asn Val Ser Ser Thr Glu Ser Gly Arg Leu Phe
                980                         985                         990

Lys Gly Lys Arg Ala His Gly Pro Ala Leu Leu Thr Lys Asp Asn Ala
        995                         1000                        1005

Leu Phe Lys Val Ser Ile Ser Leu Leu Lys Thr Asn Lys Thr Ser Asn
    1010                        1015                        1020

Asn Ser Ala Thr Asn Arg Lys Thr His Ile Asp Gly Pro Ser Leu Leu
1025                        1030                        1035                        1040

Ile Glu Asn Ser Pro Ser Val Trp Gln Asn Ile Leu Glu Ser Asp Thr
                        1045                        1050                        1055

Glu Phe Lys Lys Val Thr Pro Leu Ile His Asp Arg Met Leu Met Asp
                1060                        1065                        1070

Lys Asn Ala Thr Ala Leu Arg Leu Asn His Met Ser Asn Lys Thr Thr
        1075                        1080                        1085

Ser Ser Lys Asn Met Glu Met Val Gln Gln Lys Lys Glu Gly Pro Ile
    1090                        1095                        1100

Pro Pro Asp Ala Gln Asn Pro Asp Met Ser Phe Phe Lys Met Leu Phe
1105                        1110                        1115                        1120

Leu Pro Glu Ser Ala Arg Trp Ile Gln Arg Thr His Gly Lys Asn Ser
                        1125                        1130                        1135

Leu Asn Ser Gly Gln Gly Pro Ser Pro Lys Gln Leu Val Ser Leu Gly
                1140                        1145                        1150

Pro Glu Lys Ser Val Glu Gly Gln Asn Phe Leu Ser Glu Lys Asn Lys
        1155                        1160                        1165

Val Val Val Gly Lys Gly Glu Phe Thr Lys Asp Val Gly Leu Lys Glu
    1170                        1175                        1180

Met Val Phe Pro Ser Ser Arg Asn Leu Phe Leu Thr Asn Leu Asp Asn
1185                        1190                        1195                        1200

Leu His Glu Asn Asn Thr His Asn Gln Glu Lys Lys Ile Gln Glu Glu
                        1205                        1210                        1215

Ile Glu Lys Lys Glu Thr Leu Ile Gln Glu Asn Val Val Leu Pro Gln
                1220                        1225                        1230

Ile His Thr Val Thr Gly Thr Lys Asn Phe Met Lys Asn Leu Phe Leu
        1235                        1240                        1245

Leu Ser Thr Arg Gln Asn Val Glu Gly Ser Tyr Asp Gly Ala Tyr Ala

1250                1255                1260

Pro Val Leu Gln Asp Phe Arg Ser Leu Asn Asp Ser Thr Asn Arg Thr
1265                1270                1275                1280

Lys Lys His Thr Ala His Phe Ser Lys Lys Gly Glu Glu Glu Asn Leu
                1285                1290                1295

Glu Gly Leu Gly Asn Gln Thr Lys Gln Ile Val Glu Lys Tyr Ala Cys
            1300                1305                1310

Thr Thr Arg Ile Ser Pro Asn Thr Ser Gln Gln Asn Phe Val Thr Gln
        1315                1320                1325

Arg Ser Lys Arg Ala Leu Lys Gln Phe Arg Leu Pro Leu Glu Glu Thr
    1330                1335                1340

Glu Leu Glu Lys Arg Ile Ile Val Asp Asp Thr Ser Thr Gln Trp Ser
1345                1350                1355                1360

Lys Asn Met Lys His Leu Thr Pro Ser Thr Leu Thr Gln Ile Asp Tyr
                1365                1370                1375

Asn Glu Lys Glu Lys Gly Ala Ile Thr Gln Ser Pro Leu Ser Asp Cys
            1380                1385                1390

Leu Thr Arg Ser His Ser Ile Pro Gln Ala Asn Arg Ser Pro Leu Pro
        1395                1400                1405

Ile Ala Lys Val Ser Ser Phe Pro Ser Ile Arg Pro Ile Tyr Leu Thr
    1410                1415                1420

Arg Val Leu Phe Gln Asp Asn Ser Ser His Leu Pro Ala Ala Ser Tyr
1425                1430                1435                1440

Arg Lys Lys Asp Ser Gly Val Gln Glu Ser Ser His Phe Leu Gln Gly
                1445                1450                1455

Ala Lys Lys Asn Asn Leu Ser Leu Ala Ile Leu Thr Leu Glu Met Thr
            1460                1465                1470

Gly Asp Gln Arg Glu Val Gly Ser Leu Gly Thr Ser Ala Thr Asn Ser
        1475                1480                1485

Val Thr Tyr Lys Lys Val Glu Asn Thr Val Leu Pro Lys Pro Asp Leu
    1490                1495                1500

Pro Lys Thr Ser Gly Lys Val Glu Leu Leu Pro Lys Val His Ile Tyr
1505                1510                1515                1520

Gln Lys Asp Leu Phe Pro Thr Glu Thr Ser Asn Gly Ser Pro Gly His
                1525                1530                1535

Leu Asp Leu Val Glu Gly Ser Leu Leu Gln Gly Thr Glu Gly Ala Ile
            1540                1545                1550

Lys Trp Asn Glu Ala Asn Arg Pro Gly Lys Val Pro Phe Leu Arg Val
        1555                1560                1565

Ala Thr Glu Ser Ser Ala Lys Thr Pro Ser Lys Leu Leu Asp Pro Leu
    1570                1575                1580

Ala Trp Asp Asn His Tyr Gly Thr Gln Ile Pro Lys Glu Glu Trp Lys

1585                1590                1595                1600

Ser Gln Glu Lys Ser Pro Glu Lys Thr Ala Phe Lys Lys Lys Asp Thr
                1605                1610                1615

Ile Leu Ser Leu Asn Ala Cys Glu Ser Asn His Ala Ile Ala Ala Ile
            1620                1625                1630

Asn Glu Gly Gln Asn Lys Pro Glu Ile Glu Val Thr Trp Ala Lys Gln
        1635                1640                1645

Gly Arg Thr Glu Arg Leu Cys Ser Gln Asn Pro Pro Val Leu Lys Arg
    1650                1655                1660

His Gln Arg Glu Ile Thr Arg Thr Thr Leu Gln Ser Asp Gln Glu Glu
1665                1670                1675                1680

Ile Asp Tyr Asp Asp Thr Ile Ser Val Glu Met Lys Lys Glu Asp Phe
                1685                1690                1695

Asp Ile Tyr Asp Glu Asp Glu Asn Gln Ser Pro Arg Ser Phe Gln Lys
            1700                1705                1710

Lys Thr Arg His Tyr Phe Ile Ala Ala Val Glu Arg Leu Trp Asp Tyr
        1715                1720                1725

Gly Met Ser Ser Ser Pro His Val Leu Arg Asn Arg Ala Gln Ser Gly
    1730                1735                1740

Ser Val Pro Gln Phe Lys Lys Val Val Phe Gln Glu Phe Thr Asp Gly
1745                1750                1755                1760

Ser Phe Thr Gln Pro Leu Tyr Arg Gly Glu Leu Asn Glu His Leu Gly
                1765                1770                1775

Leu Leu Gly Pro Tyr Ile Arg Ala Glu Val Glu Asp Asn Ile Met Val
            1780                1785                1790

Thr Phe Arg Asn Gln Ala Ser Arg Pro Tyr Ser Phe Tyr Ser Ser Leu
        1795                1800                1805

Ile Ser Tyr Glu Glu Asp Gln Arg Gln Gly Ala Glu Pro Arg Lys Asn
    1810                1815                1820

Phe Val Lys Pro Asn Glu Thr Lys Thr Tyr Phe Trp Lys Val Gln His
1825                1830                1835                1840

His Met Ala Pro Thr Lys Asp Glu Phe Asp Cys Lys Ala Trp Ala Tyr
                1845                1850                1855

Phe Ser Asp Val Asp Leu Glu Lys Asp Val His Ser Gly Leu Ile Gly
            1860                1865                1870

Pro Leu Leu Val Cys His Thr Asn Thr Leu Asn Pro Ala His Gly Arg
        1875                1880                1885

Gln Val Thr Val Gln Glu Phe Ala Leu Phe Phe Thr Ile Phe Asp Glu
    1890                1895                1900

Thr Lys Ser Trp Tyr Phe Thr Glu Asn Met Glu Arg Asn Cys Arg Ala
1905                1910                1915                1920

Pro Cys Asn Ile Gln Met Glu Asp Pro Thr Phe Lys Glu Asn Tyr Arg

1925                1930                1935

Phe His Ala Ile Asn Gly Tyr Ile Met Asp Thr Leu Pro Gly Leu Val
        1940                1945                1950

Met Ala Gln Asp Gln Arg Ile Arg Trp Tyr Leu Leu Ser Met Gly Ser
    1955                1960                1965

Asn Glu Asn Ile His Ser Ile His Phe Ser Gly His Val Phe Thr Val
  1970                1975                1980

Arg Lys Lys Glu Glu Tyr Lys Met Ala Leu Tyr Asn Leu Tyr Pro Gly
1985                1990                1995                2000

Val Phe Glu Thr Val Glu Met Leu Pro Ser Lys Ala Gly Ile Trp Arg
                2005                2010                2015

Val Glu Cys Leu Ile Gly Glu His Leu His Ala Gly Met Ser Thr Leu
        2020                2025                2030

Phe Leu Val Tyr Ser Asn Lys Cys Gln Thr Pro Leu Gly Met Ala Ser
    2035                2040                2045

Gly His Ile Arg Asp Phe Gln Ile Thr Ala Ser Gly Gln Tyr Gly Gln
  2050                2055                2060

Trp Ala Pro Lys Leu Ala Arg Leu His Tyr Ser Gly Ser Ile Asn Ala
2065                2070                2075                2080

Trp Ser Thr Lys Glu Pro Phe Ser Trp Ile Lys Val Asp Leu Leu Ala
                2085                2090                2095

Pro Met Ile Ile His Gly Ile Lys Thr Gln Gly Ala Arg Gln Lys Phe
        2100                2105                2110

Ser Ser Leu Tyr Ile Ser Gln Phe Ile Ile Met Tyr Ser Leu Asp Gly
    2115                2120                2125

Lys Lys Trp Gln Thr Tyr Arg Gly Asn Ser Thr Gly Thr Leu Met Val
  2130                2135                2140

Phe Phe Gly Asn Val Asp Ser Ser Gly Ile Lys His Asn Ile Phe Asn
2145                2150                2155                2160

Pro Pro Ile Ile Ala Arg Tyr Ile Arg Leu His Pro Thr His Tyr Ser
                2165                2170                2175

Ile Arg Ser Thr Leu Arg Met Glu Leu Met Gly Cys Asp Leu Asn Ser
        2180                2185                2190

Cys Ser Met Pro Leu Gly Met Glu Ser Lys Ala Ile Ser Asp Ala Gln
    2195                2200                2205

Ile Thr Ala Ser Ser Tyr Phe Thr Asn Met Phe Ala Thr Trp Ser Pro
  2210                2215                2220

Ser Lys Ala Arg Leu His Leu Gln Gly Arg Ser Asn Ala Trp Arg Pro
2225                2230                2235                2240

Gln Val Asn Asn Pro Lys Glu Trp Leu Gln Val Asp Phe Gln Lys Thr
                2245                2250                2255

Met Lys Val Thr Gly Val Thr Thr Gln Gly Val Lys Ser Leu Leu Thr

2260                2265                2270

Ser Met Tyr Val Lys Glu Phe Leu Ile Ser Ser Ser Gln Asp Gly His
    2275                2280                2285

Gln Trp Thr Leu Phe Phe Gln Asn Gly Lys Val Lys Val Phe Gln Gly
  2290                2295                2300

Asn Gln Asp Ser Phe Thr Pro Val Val Asn Ser Leu Asp Pro Pro Leu
2305                2310                2315                2320

Leu Thr Arg Tyr Leu Arg Ile His Pro Gln Ser Trp Val His Gln Ile
                2325                2330                2335

Ala Leu Arg Met Glu Val Leu Gly Cys Glu Ala Gln Asp Leu Tyr
            2340                2345                2350

<210>   2
<211>   7056
<212>   DNA
<213>   Homo sapiens

<400>   2
atgcaaatag agctctccac ctgcttcttt ctgtgccttt tgcgattctg ctttagtgcc      60

accagaagat actacctggg tgcagtggaa ctgtcatggg actatatgca aagtgatctc     120

ggtgagctgc ctgtggacgc aagatttcct cctagagtgc caaaatcttt tccattcaac     180

acctcagtcg tgtacaaaaa gactctgttt gtagaattca cggatcacct tttcaacatc     240

gctaagccaa ggccaccctg gatgggtctg ctaggtccta ccatccaggc tgaggtttat     300

gatacagtgg tcattacact taagaacatg gcttcccatc ctgtcagtct tcatgctgtt     360

ggtgtatcct actggaaagc ttctgaggga gctgaatatg atgatcagac cagtcaaagg     420

gagaaagaag atgataaagt cttccctggt ggaagccata catatgtctg gcaggtcctg     480

aaagagaatg gtccaatggc ctctgaccca ctgtgcctta cctactcata tctttctcat     540

gtggacctgg taaaagactt gaattcaggc ctcattggag ccctactagt atgtagagaa     600

gggagtctgg ccaaggaaaa gacacagacc ttgcacaaat ttatactact ttttgctgta     660

tttgatgaag ggaaaagttg gcactcagaa acaaagaact ccttgatgca ggatagggat     720

gctgcatctg ctcgggcctg gcctaaaatg cacacagtca atggttatgt aaacaggtct     780

ctgccaggtc tgattggatg ccacaggaaa tcagtctatt ggcatgtgat tggaatgggc     840

accactcctg aagtgcactc aatattcctc gaaggtcaca catttcttgt gaggaaccat     900

cgccaggcgt ccttggaaat ctcgccaata actttcctta ctgctcaaac actcttgatg     960

gaccttggac agtttctact gttttgtcat atctcttccc accaacatga tggcatggaa    1020

gcttatgtca aagtagacag ctgtccagag gaaccccaac tacgaatgaa aaataatgaa    1080

gaagcggaag actatgatga tgatcttact gattctgaaa tggatgtggt caggtttgat    1140

gatgacaact ctccttcctt tatccaaatt cgctcagttg ccaagaagca tcctaaaact 1200

tgggtacatt acattgctgc tgaagaggag gactgggact atgctccctt agtcctcgcc 1260

cccgatgaca gaagttataa aagtcaatat ttgaacaatg gccctcagcg gattggtagg 1320

aagtacaaaa aagtccgatt tatggcatac acagatgaaa cctttaagac tcgtgaagct 1380

attcagcatg aatcaggaat cttgggacct ttactttatg gggaagttgg agacacactg 1440

ttgattatat ttaagaatca agcaagcaga ccatataaca tctaccctca cggaatcact 1500

gatgtccgtc ctttgtattc aaggagatta ccaaaaggtg taaaacattt gaaggatttt 1560

ccaattctgc caggagaaat attcaaatat aaatggacag tgactgtaga agatgggcca 1620

actaaatcag atcctcggtg cctgacccgc tattactcta gtttcgttaa tatggagaga 1680

gatctagctt caggactcat tggccctctc ctcatctgct acaaagaatc tgtagatcaa 1740

agaggaaacc agataatgtc agacaagagg aatgtcatcc tgttttctgt atttgatgag 1800

aaccgaagct ggtacctcac agagaatata caacgctttc tccccaatcc agctggagtg 1860

cagcttgagg atccagagtt ccaagcctcc aacatcatgc acagcatcaa tggctatgtt 1920

tttgatagtt tgcagttgtc agtttgtttg catgaggtgg catactggta cattctaagc 1980

attggagcac agactgactt cctttctgtc ttcttctctg gatatacctt caaacacaaa 2040

atggtctatg aagacacact caccctattc ccattctcag gagaaactgt cttcatgtcg 2100

atggaaaacc caggtctatg gattctgggg tgccacaact cagactttcg gaacagaggc 2160

atgaccgcct tactgaaggt ttctagttgt gacaagaaca ctggtgatta ttacgaggac 2220

agttatgaag atatttcagc atacttgctg agtaaaaaca atgccattga accaagaagc 2280

ttctcccaga attcaagaca ccctagcact aggcaaaagc aatttaatgc caccacaatt 2340

ccagaaaatg acatagagaa gactgaccct tggtttgcac acagaacacc tatgcctaaa 2400

atacaaaatg tctcctctag tgatttgttg atgctcttgc gacagagtcc tactccacat 2460

gggctatcct tatctgatct ccaagaagcc aaatatgaga ctttttctga tgatccatca 2520

cctggagcaa tagacagtaa taacagcctg tctgaaatga cacacttcag gccacagctc 2580

catcacagtg gggacatggt atttacccct gagtcaggcc tccaattaag attaaatgag 2640

aaactgggga caactgcagc aacagagttg aagaaacttg atttcaaagt ttctagtaca 2700

tcaaataatc tgatttcaac aattccatca gacaatttgg cagcaggtac tgataataca 2760

agttccttag gacccccaag tatgccagtt cattatgata gtcaattaga taccactcta 2820

tttggcaaaa agtcatctcc ccttactgag tctggtggac ctctgagctt gagtgaagaa 2880

aataatgatt caaagttgtt agaatcaggt ttaatgaata gccaagaaag ttcatgggga 2940

aaaaatgtat cgtcaacaga gagtggtagg ttatttaaag ggaaaagagc tcatggacct 3000

gctttgttga ctaaagataa tgccttattc aaagttagca tctctttgtt aaagacaaac 3060

aaaacttcca ataattcagc aactaataga aagactcaca ttgatggccc atcattatta 3120

attgagaata gtccatcagt ctggcaaaat atattagaaa gtgacactga gtttaaaaaa 3180

gtgacacctt tgattcatga cagaatgctt atggacaaaa atgctacagc tttgaggcta 3240

aatcatatgt caaataaaac tacttcatca aaaaacatgg aaatggtcca acagaaaaaa 3300

gagggcccca ttccaccaga tgcacaaaat ccagatatgt cgttctttaa gatgctattc 3360

ttgccagaat cagcaaggtg gatacaaagg actcatggaa agaactctct gaactctggg 3420

caaggcccca gtccaaagca attagtatcc ttaggaccag aaaaatctgt ggaaggtcag 3480

aatttcttgt ctgagaaaaa caaagtggta gtaggaaagg gtgaatttac aaaggacgta 3540

ggactcaaag agatggtttt tccaagcagc agaaacctat ttcttactaa cttggataat 3600

ttacatgaaa ataatacaca caatcaagaa aaaaaaattc aggaagaaat agaaaagaag 3660

gaaacattaa tccaagagaa tgtagttttg cctcagatac atacagtgac tggcactaag 3720

aatttcatga agaacctttt cttactgagc actaggcaaa atgtagaagg ttcatatgac 3780

ggggcatatg ctccagtact tcaagatttt aggtcattaa atgattcaac aaatagaaca 3840

aagaaacaca cagctcattt ctcaaaaaaa ggggaggaag aaaacttgga aggcttggga 3900

aatcaaacca agcaaattgt agagaaatat gcatgcacca caaggatatc tcctaataca 3960

agccagcaga attttgtcac gcaacgtagt aagagagctt tgaaacaatt cagactccca 4020

ctagaagaaa cagaacttga aaaaaggata attgtggatg acacctcaac ccagtggtcc 4080

aaaaacatga aacatttgac cccgagcacc ctcacacaga tagactacaa tgagaaggag 4140

aaaggggcca ttactcagtc tcccttatca gattgcctta cgaggagtca tagcatccct 4200

caagcaaata gatctccatt acccattgca aaggtatcat catttccatc tattagacct 4260

atatatctga ccagggtcct attccaagac aactcttctc atcttccagc agcatcttat 4320

agaaagaaag attctggggt ccaagaaagc agtcatttct tacaaggagc caaaaaaaat 4380

aacctttctt tagccattct aaccttggag atgactggtg atcaaagaga ggttggctcc 4440

ctggggacaa gtgccacaaa ttcagtcaca tacaagaaag ttgagaacac tgttctcccg 4500

aaaccagact tgcccaaaac atctggcaaa gttgaattgc ttccaaaagt tcacatttat 4560

cagaaggacc tattccctac ggaaactagc aatgggtctc ctggccatct ggatctcgtg 4620

gaagggagcc ttcttcaggg aacagaggga gcgattaagt ggaatgaagc aaacagacct 4680

ggaaaagttc cctttctgag agtagcaaca gaaagctctg caaagactcc ctccaagcta 4740

ttggatcctc ttgcttggga taaccactat ggtactcaga taccaaaaga agagtggaaa 4800

tcccaagaga agtcaccaga aaaaacagct tttaagaaaa aggataccat tttgtccctg 4860

aacgcttgtg aaagcaatca tgcaatagca gcaataaatg agggacaaaa taagcccgaa 4920

atagaagtca cctgggcaaa gcaaggtagg actgaaaggc tgtgctctca aaacccacca 4980

gtcttgaaac gccatcaacg ggaaataact cgtactactc ttcagtcaga tcaagaggaa 5040

attgactatg atgataccat atcagttgaa atgaagaagg aagattttga catttatgat 5100

gaggatgaaa atcagagccc ccgcagcttt caaaagaaaa cacgacacta ttttattgct 5160

gcagtggaga ggctctggga ttatgggatg agtagctccc cacatgttct aagaaacagg 5220

gctcagagtg gcagtgtccc tcagttcaag aaagttgttt tccaggaatt tactgatggc 5280

tcctttactc agcccttata ccgtggagaa ctaaatgaac atttgggact cctggggcca 5340

tatataagag cagaagttga agataatatc atggtaactt tcagaaatca ggcctctcgt 5400

ccctattcct tctattctag ccttatttct tatgaggaag atcagaggca aggagcagaa 5460

cctagaaaaa actttgtcaa gcctaatgaa accaaaactt acttttggaa agtgcaacat 5520

catatggcac ccactaaaga tgagtttgac tgcaaagcct gggcttattt ctctgatgtt 5580

gacctggaaa aagatgtgca ctcaggcctg attggacccc ttctggtctg ccacactaac 5640

acactgaacc ctgctcatgg gagacaagtg acagtacagg aatttgctct gtttttcacc 5700

atctttgatg agaccaaaag ctggtacttc actgaaaata tggaaagaaa ctgcagggct 5760

ccctgcaata tccagatgga agatcccact tttaaagaga attatcgctt ccatgcaatc 5820

aatggctaca taatggatac actacctggc ttagtaatgg ctcaggatca aaggattcga 5880

tggtatctgc tcagcatggg cagcaatgaa aacatccatt ctattcattt cagtggacat 5940

gtgttcactg tacgaaaaaa agaggagtat aaaatggcac tgtacaatct ctatccaggt 6000

gtttttgaga cagtggaaat gttaccatcc aaagctggaa tttggcgggt ggaatgcctt 6060

attggcgagc atctacatgc tgggatgagc acactttttc tggtgtacag caataagtgt 6120

cagactcccc tgggaatggc ttctggacac attagagatt ttcagattac agcttcagga 6180

caatatggac agtgggcccc aaagctggcc agacttcatt attccggatc aatcaatgcc 6240

tggagcacca aggagccctt ttcttggatc aaggtggatc tgttggcacc aatgattatt 6300

cacggcatca agacccaggg tgcccgtcag aagttctcca gcctctacat ctctcagttt 6360

atcatcatgt atagtcttga tgggaagaag tggcagactt atcgaggaaa ttccactgga 6420

accttaatgg tcttctttgg caatgtggat tcatctggga taaaacacaa tatttttaac 6480

cctccaatta ttgctcgata catccgtttg cacccaactc attatagcat tcgcagcact 6540

cttcgcatgg agttgatggg ctgtgattta aatagttgca gcatgccatt gggaatggag 6600

agtaaagcaa tatcagatgc acagattact gcttcatcct actttaccaa tatgtttgcc 6660

acctggtctc cttcaaaagc tcgacttcac ctccaaggga ggagtaatgc ctggagacct 6720

caggtgaata atccaaaaga gtggctgcaa gtggacttcc agaagacaat gaaagtcaca 6780

ggagtaacta ctcagggagt aaaatctctg cttaccagca tgtatgtgaa ggagttcctc 6840

atctccagca gtcaagatgg ccatcagtgg actctctttt ttcagaatgg caaagtaaag 6900

gtttttcagg gaaatcaaga ctccttcaca cctgtggtga actctctaga cccaccgtta 6960

ctgactcgct accttcgaat tcacccccag agttgggtgc accagattgc cctgaggatg 7020

gaggttctgg gctgcgaggc acaggacctc tactga 7056

<210> 3
<211> 1463
<212> PRT
<213> Artificial Sequence

<220>
<223> factor 8 mutant

<400> 3
Met Gln Ile Glu Leu Ser Thr Cys Phe Phe Leu Cys Leu Leu Arg Phe
1               5                   10                  15

Cys Phe Ser Ala Thr Arg Arg Tyr Tyr Leu Gly Ala Val Glu Leu Ser
            20                  25                  30

Trp Asp Tyr Met Gln Ser Asp Leu Gly Glu Leu Pro Val Asp Ala Arg
        35                  40                  45

Phe Pro Pro Arg Val Pro Lys Ser Phe Pro Phe Asn Thr Ser Val Val
    50                  55                  60

Tyr Lys Lys Thr Leu Phe Val Glu Phe Thr Asp His Leu Phe Asn Ile
65                  70                  75                  80

Ala Lys Pro Arg Pro Pro Trp Met Gly Leu Leu Gly Pro Thr Ile Gln
                85                  90                  95

Ala Glu Val Tyr Asp Thr Val Val Ile Thr Leu Lys Asn Met Ala Ser
            100                 105                 110

His Pro Val Ser Leu His Ala Val Gly Val Ser Tyr Trp Lys Ala Ser
        115                 120                 125

Glu Gly Ala Glu Tyr Asp Asp Gln Thr Ser Gln Arg Glu Lys Glu Asp
    130                 135                 140

Asp Lys Val Phe Pro Gly Gly Ser His Thr Tyr Val Trp Gln Val Leu
145                 150                 155                 160

Lys Glu Asn Gly Pro Met Ala Ser Asp Pro Leu Cys Leu Thr Tyr Ser
                165                 170                 175

Tyr Leu Ser His Val Asp Leu Val Lys Asp Leu Asn Ser Gly Leu Ile
            180                 185                 190

Gly Ala Leu Leu Val Cys Arg Glu Gly Ser Leu Ala Lys Glu Lys Thr
        195                 200                 205

Gln Thr Leu His Lys Phe Ile Leu Leu Phe Ala Val Phe Asp Glu Gly
    210                 215                 220

Lys Ser Trp His Ser Glu Thr Lys Asn Ser Leu Met Gln Asp Arg Asp

225                 230                 235                 240

Ala Ala Ser Ala Arg Ala Trp Pro Lys Met His Thr Val Asn Gly Tyr
                245                 250                 255

Val Asn Arg Ser Leu Pro Gly Leu Ile Gly Cys His Arg Lys Ser Val
            260                 265                 270

Tyr Trp His Val Ile Gly Met Gly Thr Thr Pro Glu Val His Ser Ile
        275                 280                 285

Phe Leu Glu Gly His Thr Phe Leu Val Arg Asn His Arg Gln Ala Ser
    290                 295                 300

Leu Glu Ile Ser Pro Ile Thr Phe Leu Thr Ala Gln Thr Leu Leu Met
305                 310                 315                 320

Asp Leu Gly Gln Phe Leu Leu Phe Cys His Ile Ser Ser His Gln His
                325                 330                 335

Asp Gly Met Glu Ala Tyr Val Lys Val Asp Ser Cys Pro Glu Glu Pro
            340                 345                 350

Gln Leu Arg Met Lys Asn Asn Glu Glu Ala Glu Asp Tyr Asp Asp Asp
        355                 360                 365

Leu Thr Asp Ser Glu Met Asp Val Val Arg Phe Asp Asp Asp Asn Ser
    370                 375                 380

Pro Ser Phe Ile Gln Ile Arg Ser Val Ala Lys Lys His Pro Lys Thr
385                 390                 395                 400

Trp Val His Tyr Ile Ala Ala Glu Glu Glu Asp Trp Asp Tyr Ala Pro
                405                 410                 415

Leu Val Leu Ala Pro Asp Asp Arg Ser Tyr Lys Ser Gln Tyr Leu Asn
            420                 425                 430

Asn Gly Pro Gln Arg Ile Gly Arg Lys Tyr Lys Lys Val Arg Phe Met
        435                 440                 445

Ala Tyr Thr Asp Glu Thr Phe Lys Thr Arg Glu Ala Ile Gln His Glu
    450                 455                 460

Ser Gly Ile Leu Gly Pro Leu Leu Tyr Gly Glu Val Gly Asp Thr Leu
465                 470                 475                 480

Leu Ile Ile Phe Lys Asn Gln Ala Ser Arg Pro Tyr Asn Ile Tyr Pro
                485                 490                 495

His Gly Ile Thr Asp Val Arg Pro Leu Tyr Ser Arg Arg Leu Pro Lys
            500                 505                 510

Gly Val Lys His Leu Lys Asp Phe Pro Ile Leu Pro Gly Glu Ile Phe
        515                 520                 525

Lys Tyr Lys Trp Thr Val Thr Val Glu Asp Gly Pro Thr Lys Ser Asp
    530                 535                 540

Pro Arg Cys Leu Thr Arg Tyr Tyr Ser Ser Phe Val Asn Met Glu Arg
545                 550                 555                 560

Asp Leu Ala Ser Gly Leu Ile Gly Pro Leu Leu Ile Cys Tyr Lys Glu

                565                 570                 575

Ser Val Asp Gln Arg Gly Asn Gln Ile Met Ser Asp Lys Arg Asn Val
            580                 585                 590

Ile Leu Phe Ser Val Phe Asp Glu Asn Arg Ser Trp Tyr Leu Thr Glu
        595                 600                 605

Asn Ile Gln Arg Phe Leu Pro Asn Pro Ala Gly Val Gln Leu Glu Asp
    610                 615                 620

Pro Glu Phe Gln Ala Ser Asn Ile Met His Ser Ile Asn Gly Tyr Val
625                 630                 635                 640

Phe Asp Ser Leu Gln Leu Ser Val Cys Leu His Glu Val Ala Tyr Trp
                645                 650                 655

Tyr Ile Leu Ser Ile Gly Ala Gln Thr Asp Phe Leu Ser Val Phe Phe
            660                 665                 670

Ser Gly Tyr Thr Phe Lys His Lys Met Val Tyr Glu Asp Thr Leu Thr
        675                 680                 685

Leu Phe Pro Phe Ser Gly Glu Thr Val Phe Met Ser Met Glu Asn Pro
    690                 695                 700

Gly Leu Trp Ile Leu Gly Cys His Asn Ser Asp Phe Arg Asn Arg Gly
705                 710                 715                 720

Met Thr Ala Leu Leu Lys Val Ser Ser Cys Asp Lys Asn Thr Gly Asp
                725                 730                 735

Tyr Tyr Glu Asp Ser Tyr Glu Asp Ile Ser Ala Tyr Leu Leu Ser Lys
            740                 745                 750

Asn Asn Ala Ile Glu Pro Arg Ser Phe Ser Gln Asn Ser Arg His Pro
        755                 760                 765

Ser Thr Arg Gln Lys Gln Phe Asn Ala Thr Thr Ile Pro Glu Asn Thr
    770                 775                 780

Thr Leu Gln Ser Asp Gln Glu Glu Ile Asp Tyr Asp Asp Thr Ile Ser
785                 790                 795                 800

Val Glu Met Lys Lys Glu Asp Phe Asp Ile Tyr Asp Glu Asp Glu Asn
                805                 810                 815

Gln Ser Pro Arg Ser Phe Gln Lys Lys Thr Arg His Tyr Phe Ile Ala
            820                 825                 830

Ala Val Glu Arg Leu Trp Asp Tyr Gly Met Ser Ser Ser Pro His Val
        835                 840                 845

Leu Arg Asn Arg Ala Gln Ser Gly Ser Val Pro Gln Phe Lys Lys Val
    850                 855                 860

Val Phe Gln Glu Phe Thr Asp Gly Ser Phe Thr Gln Pro Leu Tyr Arg
865                 870                 875                 880

Gly Glu Leu Asn Glu His Leu Gly Leu Leu Gly Pro Tyr Ile Arg Ala
                885                 890                 895

Glu Val Glu Asp Asn Ile Met Val Thr Phe Arg Asn Gln Ala Ser Arg

900                 905                 910

Pro Tyr Ser Phe Tyr Ser Ser Leu Ile Ser Tyr Glu Glu Asp Gln Arg
        915                 920                 925

Gln Gly Ala Glu Pro Arg Lys Asn Phe Val Lys Pro Asn Glu Thr Lys
    930                 935                 940

Thr Tyr Phe Trp Lys Val Gln His His Met Ala Pro Thr Lys Asp Glu
945                 950                 955                 960

Phe Asp Cys Lys Ala Trp Ala Tyr Phe Ser Asp Val Asp Leu Glu Lys
                965                 970                 975

Asp Val His Ser Gly Leu Ile Gly Pro Leu Leu Val Cys His Thr Asn
            980                 985                 990

Thr Leu Asn Pro Ala His Gly Arg Gln Val Thr Val Gln Glu Phe Ala
        995                 1000                1005

Leu Phe Phe Thr Ile Phe Asp Glu Thr Lys Ser Trp Tyr Phe Thr Glu
    1010                1015                1020

Asn Met Glu Arg Asn Cys Arg Ala Pro Cys Asn Ile Gln Met Glu Asp
1025                1030                1035                1040

Pro Thr Phe Lys Glu Asn Tyr Arg Phe His Ala Ile Asn Gly Tyr Ile
                1045                1050                1055

Met Asp Thr Leu Pro Gly Leu Val Met Ala Gln Asp Gln Arg Ile Arg
            1060                1065                1070

Trp Tyr Leu Leu Ser Met Gly Ser Asn Glu Asn Ile His Ser Ile His
        1075                1080                1085

Phe Ser Gly His Val Phe Thr Val Arg Lys Lys Glu Glu Tyr Lys Met
    1090                1095                1100

Ala Leu Tyr Asn Leu Tyr Pro Gly Val Phe Glu Thr Val Glu Met Leu
1105                1110                1115                1120

Pro Ser Lys Ala Gly Ile Trp Arg Val Glu Cys Leu Ile Gly Glu His
                1125                1130                1135

Leu His Ala Gly Met Ser Thr Leu Phe Leu Val Tyr Ser Asn Lys Cys
            1140                1145                1150

Gln Thr Pro Leu Gly Met Ala Ser Gly His Ile Arg Asp Phe Gln Ile
        1155                1160                1165

Thr Ala Ser Gly Gln Tyr Gly Gln Trp Ala Pro Lys Leu Ala Arg Leu
    1170                1175                1180

His Tyr Ser Gly Ser Ile Asn Ala Trp Ser Thr Lys Glu Pro Phe Ser
1185                1190                1195                1200

Trp Ile Lys Val Asp Leu Leu Ala Pro Met Ile Ile His Gly Ile Lys
                1205                1210                1215

Thr Gln Gly Ala Arg Gln Lys Phe Ser Ser Leu Tyr Ile Ser Gln Phe
            1220                1225                1230

Ile Ile Met Tyr Ser Leu Asp Gly Lys Lys Trp Gln Thr Tyr Arg Gly

1235                1240                1245

Asn Ser Thr Gly Thr Leu Met Val Phe Phe Gly Asn Val Asp Ser Ser
    1250                1255                1260

Gly Ile Lys His Asn Ile Phe Asn Pro Pro Ile Ile Ala Arg Tyr Ile
1265                1270                1275                1280

Arg Leu His Pro Thr His Tyr Ser Ile Arg Ser Thr Leu Arg Met Glu
                1285                1290                1295

Leu Met Gly Cys Asp Leu Asn Ser Cys Ser Met Pro Leu Gly Met Glu
            1300                1305                1310

Ser Lys Ala Ile Ser Asp Ala Gln Ile Thr Ala Ser Ser Tyr Phe Thr
        1315                1320                1325

Asn Met Phe Ala Thr Trp Ser Pro Ser Lys Ala Arg Leu His Leu Gln
    1330                1335                1340

Gly Arg Ser Asn Ala Trp Arg Pro Gln Val Asn Asn Pro Lys Glu Trp
1345                1350                1355                1360

Leu Gln Val Asp Phe Gln Lys Thr Met Lys Val Thr Gly Val Thr Thr
                1365                1370                1375

Gln Gly Val Lys Ser Leu Leu Thr Ser Met Tyr Val Lys Glu Phe Leu
            1380                1385                1390

Ile Ser Ser Ser Gln Asp Gly His Gln Trp Thr Leu Phe Phe Gln Asn
        1395                1400                1405

Gly Lys Val Lys Val Phe Gln Gly Asn Gln Asp Ser Phe Thr Pro Val
    1410                1415                1420

Val Asn Ser Leu Asp Pro Pro Leu Leu Thr Arg Tyr Leu Arg Ile His
1425                1430                1435                1440

Pro Gln Ser Trp Val His Gln Ile Ala Leu Arg Met Glu Val Leu Gly
                1445                1450                1455

Cys Glu Ala Gln Asp Leu Tyr
            1460


<210>   4
<211>   4392
<212>   PRT
<213>   Artificial Sequence

<220>
<223>   factor 8 mutant CO


<400>   4
   Ala Thr Gly Cys Ala Gly Ala Thr Thr Gly Ala Gly Cys Thr Gly Thr
   1               5                   10                  15
   Cys Cys Ala Cys Cys Thr Gly Thr Thr Thr Cys Thr Thr Cys Cys Thr
               20                  25                  30
   Gly Thr Gly Cys Cys Thr Gly Cys Thr Gly Ala Gly Ala Thr Thr Thr
           35                  40                  45
   Thr Gly Cys Thr Thr Thr Thr Cys Cys Gly Cys Cys Ala Cys Thr Cys
        50                  55                  60
   Gly Cys Cys Gly Ala Thr Ala Cys Thr Ala Cys Cys Thr Gly Gly Gly
    65                  70                  75                  80
   Gly Gly Cys Thr Gly Thr Gly Gly Ala Gly Cys Thr Gly Ala Gly Cys
                    85                  90                  95
   Thr Gly Gly Gly Ala Thr Thr Ala Cys Ala Thr Gly Cys Ala Gly Thr
               100                 105                 110
   Cys Cys Gly Ala Cys Cys Thr Gly Gly Gly Ala Gly Ala Gly Cys Thr
           115                 120                 125
   Gly Cys Cys Ala Gly Thr Gly Gly Ala Cys Gly Cys Ala Ala Gly Gly
       130                 135                 140
   Thr Thr Thr Cys Cys Ala Cys Cys Thr Cys Gly Cys Gly Thr Gly Cys
   145                 150                 155                 160
   Cys Ala Ala Ala Gly Thr Cys Thr Thr Thr Cys Cys Cys Cys Thr Thr
                   165                 170                 175
   Thr Ala Ala Thr Ala Cys Ala Ala Gly Cys Gly Thr Gly Gly Thr Gly
               180                 185                 190
   Thr Ala Thr Ala Ala Gly Ala Ala Ala Ala Cys Cys Cys Thr Gly Thr
           195                 200                 205
   Thr Cys Gly Thr Gly Gly Ala Gly Thr Thr Thr Ala Cys Ala Gly Ala
       210                 215                 220
   Thr Cys Ala Cys Cys Thr Gly Thr Thr Cys Ala Ala Cys Ala Thr Cys
   225                 230                 235                 240
   Gly Cys Cys Ala Ala Gly Cys Cys Ala Ala Gly Gly Cys Cys Ala Cys
                   245                 250                 255
   Cys Cys Thr Gly Gly Ala Thr Gly Gly Gly Cys Cys Thr Gly Cys Thr
               260                 265                 270
   Gly Gly Gly Ala Cys Cys Ala Ala Cys Cys Ala Thr Cys Cys Ala Gly
           275                 280                 285
   Gly Cys Ala Gly Ala Gly Gly Thr Gly Thr Ala Cys Gly Ala Cys Ala
       290                 295                 300
   Cys Cys Gly Thr Gly Gly Thr Cys Ala Thr Cys Ala Cys Cys Cys Thr
   305                 310                 315                 320
   Gly Ala Ala Gly Ala Ala Cys Ala Thr Gly Gly Cys Cys Thr Cys Thr
                   325                 330                 335
   Cys Ala Cys Cys Cys Cys Gly Thr Gly Ala Gly Cys Cys Thr Gly Cys
               340                 345                 350
   Ala Cys Gly Cys Ala Gly Thr Gly Gly Gly Cys Gly Thr Gly Thr Cys
           355                 360                 365
   Thr Thr Ala Cys Thr Gly Gly Ala Ala Gly Gly Cys Cys Ala Gly Cys
       370                 375                 380
   Gly Ala Gly Gly Gly Cys Gly Cys Cys Gly Ala Gly Thr Ala Thr Gly
   385                 390                 395                 400
   Ala Cys Gly Ala Thr Cys Ala Gly Ala Cys Cys Ala Gly Cys Cys Ala
                   405                 410                 415
   Gly Cys Gly Cys Gly Ala Gly Ala Ala Gly Gly Ala Gly Gly Ala Cys
               420                 425                 430
   Gly Ala Thr Ala Ala Gly Gly Thr Gly Thr Thr Thr Cys Cys Thr Gly
           435                 440                 445
   Gly Cys Gly Gly Cys Thr Cys Cys Cys Ala Cys Ala Cys Cys Thr Ala
       450                 455                 460
   Cys Gly Thr Gly Thr Gly Gly Cys Ala Gly Gly Thr Gly Cys Thr Gly
   465                 470                 475                 480
   Ala Ala Gly Gly Ala Gly Ala Ala Thr Gly Gly Cys Cys Cys Thr Ala
                   485                 490                 495
   Thr Gly Gly Cys Cys Ala Gly Cys Gly Ala Cys Cys Cys Ala Cys Thr
               500                 505                 510
   Gly Thr Gly Cys Cys Thr Gly Ala Cys Cys Thr Ala Cys Thr Cys Thr
           515                 520                 525
   Thr Ala Thr Cys Thr Gly Ala Gly Cys Cys Ala Cys Gly Thr Gly Gly
       530                 535                 540
   Ala Cys Cys Thr Gly Gly Thr Gly Ala Ala Gly Gly Ala Thr Cys Thr
   545                 550                 555                 560
   Gly Ala Ala Cys Thr Cys Cys Gly Gly Cys Cys Thr Gly Ala Thr Cys
                   565                 570                 575
   Gly Gly Cys Gly Cys Cys Cys Thr Gly Cys Thr Gly Gly Thr Gly Thr
               580                 585                 590
   Gly Cys Ala Gly Ala Gly Ala Gly Gly Gly Cys Thr Cys Thr Cys Thr
           595                 600                 605
   Gly Gly Cys Cys Ala Ala Gly Gly Ala Gly Ala Ala Ala Ala Cys Cys
       610                 615                 620
   Cys Ala Gly Ala Cys Cys Cys Thr Gly Cys Ala Cys Ala Ala Gly Thr
   625                 630                 635                 640
   Thr Cys Ala Thr Cys Cys Thr Gly Cys Thr Gly Thr Thr Cys Gly Cys
                   645                 650                 655
   Cys Gly Thr Gly Thr Thr Thr Gly Ala Cys Gly Ala Gly Gly Gly Cys
               660                 665                 670
   Ala Ala Gly Thr Cys Cys Thr Gly Gly Cys Ala Cys Ala Gly Cys Gly
           675                 680                 685
   Ala Gly Ala Cys Ala Ala Ala Gly Ala Ala Cys Ala Gly Cys Cys Thr
       690                 695                 700
   Gly Ala Thr Gly Cys Ala Gly Gly Ala Cys Ala Gly Gly Gly Ala Thr
   705                 710                 715                 720
   Gly Cys Ala Gly Cys Cys Thr Cys Thr Gly Cys Cys Cys Gly Cys Gly
                   725                 730                 735
   Cys Cys Thr Gly Gly Cys Cys Cys Ala Ala Gly Ala Thr Gly Cys Ala
               740                 745                 750
   Cys Ala Cys Ala Gly Thr Gly Ala Ala Cys Gly Gly Cys Thr Ala Cys
           755                 760                 765
   Gly Thr Gly Ala Ala Thr Ala Gly Gly Thr Cys Cys Cys Thr Gly Cys
       770                 775                 780
   Cys Thr Gly Gly Cys Cys Thr Gly Ala Thr Cys Gly Gly Ala Thr Gly
   785                 790                 795                 800
   Cys Cys Ala Cys Cys Gly Cys Ala Ala Gly Ala Gly Cys Gly Thr Gly
                   805                 810                 815
   Thr Ala Cys Thr Gly Gly Cys Ala Cys Gly Thr Gly Ala Thr Cys Gly
               820                 825                 830
   Gly Cys Ala Thr Gly Gly Gly Cys Ala Cys Cys Ala Cys Ala Cys Cys
           835                 840                 845
   Thr Gly Ala Gly Gly Thr Gly Cys Ala Cys Thr Cys Cys Ala Thr Cys
       850                 855                 860
   Thr Thr Cys Cys Thr Gly Gly Ala Gly Gly Gly Cys Cys Ala Cys Ala
   865                 870                 875                 880
   Cys Cys Thr Thr Thr Cys Thr Gly Gly Thr Gly Cys Gly Gly Ala Ala
                   885                 890                 895
   Cys Cys Ala Cys Ala Gly Ala Cys Ala Gly Gly Cys Cys Ala Gly Cys
               900                 905                 910
   Cys Thr Gly Gly Ala Gly Ala Thr Cys Ala Gly Cys Cys Cys Ala Ala
           915                 920                 925
   Thr Cys Ala Cys Cys Thr Thr Cys Cys Thr Gly Ala Cys Ala Gly Cys
       930                 935                 940
   Cys Cys Ala Gly Ala Cys Cys Cys Thr Gly Cys Thr Gly Ala Thr Gly
   945                 950                 955                 960
   Gly Ala Thr Cys Thr Gly Gly Gly Cys Cys Ala Gly Thr Thr Cys Cys
                   965                 970                 975
   Thr Gly Cys Thr Gly Thr Thr Thr Thr Gly Cys Cys Ala Cys Ala Thr
               980                 985                 990
   Cys Ala Gly Cys Thr Cys Cys Cys Ala Cys Cys Ala Gly Cys Ala Cys
           995                 1000                1005
   Gly Ala Thr Gly Gly Cys Ala Thr Gly Gly Ala Gly Gly Cys Cys Thr
       1010                1015                1020
   Ala Cys Gly Thr Gly Ala Ala Gly Gly Thr Gly Gly Ala Cys Ala Gly
   1025                1030                1035                1040
   Cys Thr Gly Thr Cys Cys Thr Gly Ala Gly Gly Ala Gly Cys Cys Ala
                   1045                1050                1055
   Cys Ala Gly Cys Thr Gly Cys Gly Gly Ala Thr Gly Ala Ala Gly Ala
   1060 1065 1070
   Ala Cys Ala Ala Thr Gly Ala Gly Gly Ala Gly Gly Cys Cys Gly Ala
   1075 1080 1085
   Gly Gly Ala Cys Thr Ala Thr Gly Ala Cys Gly Ala Thr Gly Ala Cys
   1090 1095 1100
   Cys Thr Gly Ala Cys Ala Gly Ala Cys Thr Cys Cys Gly Ala Gly Ala
   1105 1110 1115 1120
   Thr Gly Gly Ala Thr Gly Thr Gly Gly Thr Gly Cys Gly Gly Thr Thr
   1125 1130 1135
   Cys Gly Ala Thr Gly Ala Cys Gly Ala Thr Ala Ala Cys Thr Cys Thr
   1140 1145 1150
   Cys Cys Cys Ala Gly Cys Thr Thr Thr Ala Thr Cys Cys Ala Gly Ala
   1155 1160 1165
   Thr Cys Cys Gly Gly Ala Gly Cys Gly Thr Gly Gly Cys Cys Ala Ala
   1170 1175 1180
   Gly Ala Ala Gly Cys Ala Cys Cys Cys Thr Ala Ala Gly Ala Cys Cys
   1185 1190 1195 1200
   Thr Gly Gly Gly Thr Gly Cys Ala Cys Thr Ala Cys Ala Thr Cys Gly
   1205 1210 1215
   Cys Cys Gly Cys Cys Gly Ala Gly Gly Ala Gly Gly Ala Gly Gly Ala
   1220 1225 1230
   Cys Thr Gly Gly Gly Ala Thr Thr Ala Thr Gly Cys Cys Cys Cys Cys
   1235 1240 1245
   Cys Thr Gly Gly Thr Gly Cys Thr Gly Gly Cys Cys Cys Cys Thr Gly
   1250 1255 1260
   Ala Cys Gly Ala Thr Ala Gly Ala Ala Gly Cys Thr Ala Cys Ala Ala
   1265 1270 1275 1280
   Gly Thr Cys Cys Cys Ala Gly Thr Ala Thr Cys Thr Gly Ala Ala Cys
   1285 1290 1295
   Ala Ala Thr Gly Gly Cys Cys Cys Ala Cys Ala Gly Ala Gly Gly Ala
   1300 1305 1310
   Thr Cys Gly Gly Cys Cys Gly Cys Ala Ala Gly Thr Ala Cys Ala Ala
   1315 1320 1325
   Gly Ala Ala Gly Gly Thr Gly Ala Gly Gly Thr Thr Cys Ala Thr Gly
   1330 1335 1340
   Gly Cys Cys Thr Ala Thr Ala Cys Ala Gly Ala Cys Gly Ala Gly Ala
   1345 1350 1355 1360
   Cys Ala Thr Thr Cys Ala Ala Gly Ala Cys Ala Cys Gly Cys Gly Ala
   1365 1370 1375
   Gly Gly Cys Cys Ala Thr Cys Cys Ala Gly Cys Ala Cys Gly Ala Gly
   1380 1385 1390
   Ala Gly Cys Gly Gly Cys Ala Thr Cys Cys Thr Gly Gly Gly Ala Cys
           1395                1400                1405
   Cys Ala Cys Thr Gly Cys Thr Gly Thr Ala Cys Gly Gly Ala Gly Ala
       1410                1415                1420
   Ala Gly Thr Gly Gly Gly Cys Gly Ala Cys Ala Cys Cys Cys Thr Gly
   1425                1430                1435                1440
   Cys Thr Gly Ala Thr Cys Ala Thr Cys Thr Thr Thr Ala Ala Gly Ala
                   1445                1450                1455
   Ala Cys Cys Ala Gly Gly Cys Cys Thr Cys Thr Ala Gly Gly Cys Cys
               1460                1465                1470
   Ala Thr Ala Cys Ala Ala Thr Ala Thr Cys Thr Ala Thr Cys Cys Cys
           1475                1480                1485
   Cys Ala Cys Gly Gly Cys Ala Thr Cys Ala Cys Ala Gly Ala Thr Gly
       1490                1495                1500
   Thr Gly Cys Gly Cys Cys Cys Ala Cys Thr Gly Thr Ala Cys Ala Gly
   1505                1510                1515                1520
   Cys Cys Gly Gly Ala Gly Ala Cys Thr Gly Cys Cys Cys Ala Ala Gly
                   1525                1530                1535
   Gly Gly Cys Gly Thr Gly Ala Ala Gly Cys Ala Cys Cys Thr Gly Ala
               1540                1545                1550
   Ala Gly Gly Ala Cys Thr Thr Cys Cys Cys Cys Ala Thr Cys Cys Thr
           1555                1560                1565
   Gly Cys Cys Thr Gly Gly Cys Gly Ala Gly Ala Thr Thr Thr Thr Cys
       1570                1575                1580
   Ala Ala Gly Thr Ala Thr Ala Ala Gly Thr Gly Gly Ala Cys Cys Gly
   1585                1590                1595                1600
   Thr Gly Ala Cys Ala Gly Thr Gly Gly Ala Gly Gly Ala Thr Gly Gly
                   1605                1610                1615
   Cys Cys Cys Thr Ala Cys Cys Ala Ala Gly Thr Cys Cys Gly Ala Cys
               1620                1625                1630
   Cys Cys Ala Cys Gly Gly Thr Gly Cys Cys Thr Gly Ala Cys Ala Ala
           1635                1640                1645
   Gly Ala Thr Ala Cys Thr Ala Thr Thr Cys Thr Ala Gly Cys Thr Thr
       1650                1655                1660
   Cys Gly Thr Gly Ala Ala Thr Ala Thr Gly Gly Ala Gly Cys Gly Gly
   1665                1670                1675                1680
   Gly Ala Thr Cys Thr Gly Gly Cys Cys Ala Gly Cys Gly Gly Cys Cys
                   1685                1690                1695
   Thr Gly Ala Thr Cys Gly Gly Ala Cys Cys Ala Cys Thr Gly Cys Thr
               1700                1705                1710
   Gly Ala Thr Cys Thr Gly Thr Thr Ala Cys Ala Ala Gly Gly Ala Gly
           1715                1720                1725
   Ala Gly Cys Gly Thr Gly Gly Ala Thr Cys Ala Gly Cys Gly Gly Gly
   1730                1735                1740
   Gly Cys Ala Ala Cys Cys Ala Gly Ala Thr Cys Ala Thr Gly Thr Cys
   1745                1750                1755                1760
   Cys Gly Ala Cys Ala Ala Gly Ala Gly Ala Ala Ala Thr Gly Thr Gly
                   1765                1770                1775
   Ala Thr Cys Cys Thr Gly Thr Thr Cys Ala Gly Cys Gly Thr Gly Thr
               1780                1785                1790
   Thr Thr Gly Ala Cys Gly Ala Gly Ala Ala Cys Cys Gly Gly Thr Cys
           1795                1800                1805
   Cys Thr Gly Gly Thr Ala Thr Cys Thr Gly Ala Cys Cys Gly Ala Gly
       1810                1815                1820
   Ala Ala Cys Ala Thr Cys Cys Ala Gly Ala Gly Ala Thr Thr Cys Cys
   1825                1830                1835                1840
   Thr Gly Cys Cys Ala Ala Ala Thr Cys Cys Ala Gly Cys Ala Gly Gly
                   1845                1850                1855
   Ala Gly Thr Gly Cys Ala Gly Cys Thr Gly Gly Ala Gly Gly Ala Cys
               1860                1865                1870
   Cys Cys Cys Gly Ala Gly Thr Thr Thr Cys Ala Gly Gly Cys Cys Thr
           1875                1880                1885
   Cys Cys Ala Ala Cys Ala Thr Cys Ala Thr Gly Cys Ala Cys Thr Cys
       1890                1895                1900
   Thr Ala Thr Cys Ala Ala Thr Gly Gly Cys Thr Ala Cys Gly Thr Gly
   1905                1910                1915                1920
   Thr Thr Cys Gly Ala Cys Ala Gly Cys Cys Thr Gly Cys Ala Gly Cys
                   1925                1930                1935
   Thr Gly Ala Gly Cys Gly Thr Gly Thr Gly Cys Cys Thr Gly Cys Ala
               1940                1945                1950
   Cys Gly Ala Gly Gly Thr Gly Gly Cys Cys Thr Ala Cys Thr Gly Gly
           1955                1960                1965
   Thr Ala Thr Ala Thr Cys Cys Thr Gly Thr Cys Cys Ala Thr Cys Gly
       1970                1975                1980
   Gly Cys Gly Cys Cys Cys Ala Gly Ala Cys Cys Gly Ala Thr Thr Thr
   1985                1990                1995                2000
   Cys Cys Thr Gly Ala Gly Cys Gly Thr Gly Thr Thr Cys Thr Thr Cys
                   2005                2010                2015
   Ala Gly Cys Gly Gly Cys Thr Ala Cys Ala Cys Cys Thr Thr Cys Ala
               2020                2025                2030
   Ala Gly Cys Ala Cys Ala Ala Gly Ala Thr Gly Gly Thr Gly Thr Ala
           2035                2040                2045
   Thr Gly Ala Gly Gly Ala Cys Ala Cys Cys Cys Thr Gly Ala Cys Ala
       2050                2055                2060
   Cys Thr Gly Thr Thr Cys Cys Cys Ala Thr Thr Thr Thr Cys Thr Gly
   2065                2070                2075                2080
   Gly Cys Gly Ala Gly Ala Cys Ala Gly Thr Gly Thr Thr Cys Ala Thr
                   2085                2090                2095
   Gly Ala Gly Cys Ala Thr Gly Gly Ala Gly Ala Ala Thr Cys Cys Cys
               2100                2105                2110
   Gly Gly Cys Cys Thr Gly Thr Gly Gly Ala Thr Thr Cys Thr Gly Gly
           2115                2120                2125
   Gly Cys Thr Gly Cys Cys Ala Cys Ala Ala Cys Ala Gly Cys Gly Ala
       2130                2135                2140
   Thr Thr Thr Cys Ala Gly Gly Ala Ala Thr Cys Gly Cys Gly Gly Cys
   2145                2150                2155                2160
   Ala Thr Gly Ala Cys Cys Gly Cys Cys Cys Thr Gly Cys Thr Gly Ala
                   2165                2170                2175
   Ala Gly Gly Thr Gly Thr Cys Cys Thr Cys Thr Thr Gly Thr Gly Ala
               2180                2185                2190
   Cys Ala Ala Gly Ala Ala Cys Ala Cys Ala Gly Gly Cys Gly Ala Cys
           2195                2200                2205
   Thr Ala Cys Thr Ala Thr Gly Ala Gly Gly Ala Thr Ala Gly Cys Thr
       2210                2215                2220
   Ala Cys Gly Ala Gly Gly Ala Cys Ala Thr Cys Thr Cys Cys Gly Cys
   2225                2230                2235                2240
   Cys Thr Ala Thr Cys Thr Gly Cys Thr Gly Thr Cys Thr Ala Ala Gly
                   2245                2250                2255
   Ala Ala Cys Ala Ala Thr Gly Cys Cys Ala Thr Cys Gly Ala Gly Cys
               2260                2265                2270
   Cys Thr Ala Gly Gly Thr Cys Cys Thr Thr Thr Thr Cys Thr Cys Ala
           2275                2280                2285
   Gly Ala Ala Cys Ala Gly Cys Cys Gly Gly Cys Ala Cys Cys Cys Ala
       2290                2295                2300
   Thr Cys Thr Ala Cys Cys Ala Gly Ala Cys Ala Gly Ala Ala Gly Cys
   2305                2310                2315                2320
   Ala Gly Thr Thr Cys Ala Ala Cys Gly Cys Cys Ala Cys Cys Ala Cys
                   2325                2330                2335
   Ala Ala Thr Cys Cys Cys Thr Gly Ala Gly Ala Ala Thr Ala Cys Cys
               2340                2345                2350
   Ala Cys Ala Cys Thr Gly Cys Ala Gly Thr Cys Thr Gly Ala Thr Cys
           2355                2360                2365
   Ala Gly Gly Ala Gly Gly Ala Gly Ala Thr Cys Gly Ala Cys Thr Ala
       2370                2375                2380
   Cys Gly Ala Cys Gly Ala Thr Ala Cys Ala Ala Thr Cys Ala Gly Cys
   2385                2390                2395                2400
   Gly Thr Gly Gly Ala Gly Ala Thr Gly Ala Ala Gly Ala Ala Gly Gly
                   2405                2410                2415
   Ala Gly Gly Ala Cys Thr Thr Cys Gly Ala Cys Ala Thr Cys Thr Ala
               2420                2425                2430
   Cys Gly Ala Cys Gly Ala Gly Gly Ala Thr Gly Ala Gly Ala Ala Cys
           2435                2440                2445
   Cys Ala Gly Thr Cys Thr Cys Cys Ala Ala Gly Gly Ala Gly Cys Thr
       2450                2455                2460
   Thr Cys Cys Ala Gly Ala Ala Gly Ala Ala Ala Ala Cys Cys Cys Gly
   2465                2470                2475                2480
   Gly Cys Ala Cys Thr Ala Cys Thr Thr Thr Ala Thr Cys Gly Cys Cys
                   2485                2490                2495
   Gly Cys Cys Gly Thr Gly Gly Ala Gly Cys Gly Cys Cys Thr Gly Thr
               2500                2505                2510
   Gly Gly Gly Ala Thr Thr Ala Thr Gly Gly Cys Ala Thr Gly Ala Gly
           2515                2520                2525
   Cys Ala Gly Cys Ala Gly Cys Cys Cys Thr Cys Ala Cys Gly Thr Gly
       2530                2535                2540
   Cys Thr Gly Cys Gly Gly Ala Ala Thr Ala Gly Ala Gly Cys Cys Cys
   2545                2550                2555                2560
   Ala Gly Ala Gly Cys Gly Gly Cys Thr Cys Cys Gly Thr Gly Cys Cys
                   2565                2570                2575
   Ala Cys Ala Gly Thr Thr Cys Ala Ala Gly Ala Ala Gly Gly Thr Gly
               2580                2585                2590
   Gly Thr Gly Thr Thr Cys Cys Ala Gly Gly Ala Gly Thr Thr Thr Ala
           2595                2600                2605
   Cys Cys Gly Ala Cys Gly Gly Cys Thr Cys Cys Thr Thr Thr Ala Cys
       2610                2615                2620
   Ala Cys Ala Gly Cys Cys Ala Cys Thr Gly Thr Ala Cys Ala Gly Gly
   2625                2630                2635                2640
   Gly Gly Ala Gly Ala Gly Cys Thr Gly Ala Ala Cys Gly Ala Gly Cys
                   2645                2650                2655
   Ala Cys Cys Thr Gly Gly Gly Cys Cys Thr Gly Cys Thr Gly Gly Gly
               2660                2665                2670
   Ala Cys Cys Cys Thr Ala Thr Ala Thr Cys Ala Gly Ala Gly Cys Cys
           2675                2680                2685
   Gly Ala Gly Gly Thr Gly Gly Ala Gly Gly Ala Thr Ala Ala Cys Ala
       2690                2695                2700
   Thr Cys Ala Thr Gly Gly Thr Gly Ala Cys Cys Thr Thr Cys Ala Gly
   2705                2710                2715                2720
   Gly Ala Ala Thr Cys Ala Gly Gly Cys Cys Ala Gly Cys Cys Gly Cys
                   2725                2730                2735
   Cys Cys Cys Thr Ala Cys Thr Cys Cys Thr Thr Thr Thr Ala Thr Ala
   2740 2745 2750
   Gly Cys Thr Cys Cys Cys Thr Gly Ala Thr Cys Ala Gly Cys Thr Ala
   2755 2760 2765
   Cys Gly Ala Gly Gly Ala Gly Gly Ala Cys Cys Ala Gly Ala Gly Gly
   2770 2775 2780
   Cys Ala Gly Gly Gly Ala Gly Cys Ala Gly Ala Gly Cys Cys Thr Ala
   2785 2790 2795 2800
   Gly Ala Ala Ala Gly Ala Ala Cys Thr Thr Cys Gly Thr Gly Ala Ala
   2805 2810 2815
   Gly Cys Cys Ala Ala Ala Cys Gly Ala Gly Ala Cys Ala Ala Ala Gly
   2820 2825 2830
   Ala Cys Cys Thr Ala Cys Thr Thr Thr Thr Gly Gly Ala Ala Gly Gly
   2835 2840 2845
   Thr Gly Cys Ala Gly Cys Ala Cys Cys Ala Cys Ala Thr Gly Gly Cys
   2850 2855 2860
   Cys Cys Cys Thr Ala Cys Cys Ala Ala Gly Gly Ala Cys Gly Ala Gly
   2865 2870 2875 2880
   Thr Thr Cys Gly Ala Thr Thr Gly Cys Ala Ala Gly Gly Cys Cys Thr
   2885 2890 2895
   Gly Gly Gly Cys Cys Thr Ala Thr Thr Thr Thr Thr Cys Thr Gly Ala
   2900 2905 2910
   Cys Gly Thr Gly Gly Ala Thr Cys Thr Gly Gly Ala Gly Ala Ala Gly
   2915 2920 2925
   Gly Ala Cys Gly Thr Gly Cys Ala Thr Thr Cys Cys Gly Gly Cys Cys
   2930 2935 2940
   Thr Gly Ala Thr Cys Gly Gly Cys Cys Cys Thr Cys Thr Gly Cys Thr
   2945 2950 2955 2960
   Gly Gly Thr Gly Thr Gly Cys Cys Ala Cys Ala Cys Cys Ala Ala Cys
   2965 2970 2975
   Ala Cys Ala Cys Thr Gly Ala Ala Thr Cys Cys Ala Gly Cys Cys Cys
   2980 2985 2990
   Ala Cys Gly Gly Cys Cys Gly Gly Cys Ala Gly Gly Thr Cys Ala Cys
   2995 3000 3005
   Cys Gly Thr Gly Cys Ala Gly Gly Ala Gly Thr Thr Cys Gly Cys Cys
   3010 3015 3020
   Cys Thr Gly Thr Thr Cys Thr Thr Thr Ala Cys Cys Ala Thr Cys Thr
   3025 3030 3035 3040
   Thr Thr Gly Ala Thr Gly Ala Gly Ala Cys Ala Ala Ala Gly Thr Cys
   3045 3050 3055
   Cys Thr Gly Gly Thr Ala Cys Thr Thr Cys Ala Cys Ala Gly Ala Gly
   3060 3065 3070
   Ala Ala Cys Ala Thr Gly Gly Ala Gly Cys Gly Gly Ala Ala Thr Thr
           3075                3080                3085
   Gly Cys Ala Gly Ala Gly Cys Cys Cys Cys Cys Thr Gly Thr Ala Ala
       3090                3095                3100
   Thr Ala Thr Cys Cys Ala Gly Ala Thr Gly Gly Ala Gly Gly Ala Cys
   3105                3110                3115                3120
   Cys Cys Thr Ala Cys Cys Thr Thr Cys Ala Ala Gly Gly Ala Gly Ala
                   3125                3130                3135
   Ala Cys Thr Ala Cys Ala Gly Ala Thr Thr Thr Cys Ala Cys Gly Cys
               3140                3145                3150
   Cys Ala Thr Cys Ala Ala Thr Gly Gly Cys Thr Ala Thr Ala Thr Cys
           3155                3160                3165
   Ala Thr Gly Gly Ala Thr Ala Cys Ala Cys Thr Gly Cys Cys Ala Gly
       3170                3175                3180
   Gly Cys Cys Thr Gly Gly Thr Cys Ala Thr Gly Gly Cys Ala Cys Ala
   3185                3190                3195                3200
   Gly Gly Ala Cys Cys Ala Gly Cys Gly Gly Ala Thr Cys Ala Gly Ala
                   3205                3210                3215
   Thr Gly Gly Thr Ala Thr Cys Thr Gly Cys Thr Gly Ala Gly Cys Ala
               3220                3225                3230
   Thr Gly Gly Gly Cys Ala Gly Cys Ala Ala Cys Gly Ala Gly Ala Ala
           3235                3240                3245
   Thr Ala Thr Cys Cys Ala Cys Thr Cys Thr Ala Thr Cys Cys Ala Cys
       3250                3255                3260
   Thr Thr Cys Ala Gly Cys Gly Gly Cys Cys Ala Cys Gly Thr Gly Thr
   3265                3270                3275                3280
   Thr Thr Ala Cys Cys Gly Thr Gly Ala Gly Gly Ala Ala Gly Ala Ala
                   3285                3290                3295
   Gly Gly Ala Gly Gly Ala Gly Thr Ala Thr Ala Ala Gly Ala Thr Gly
               3300                3305                3310
   Gly Cys Cys Cys Thr Gly Thr Ala Cys Ala Ala Cys Cys Thr Gly Thr
           3315                3320                3325
   Ala Thr Cys Cys Cys Gly Gly Cys Gly Thr Gly Thr Thr Cys Gly Ala
       3330                3335                3340
   Gly Ala Cys Ala Gly Thr Gly Gly Ala Gly Ala Thr Gly Cys Thr Gly
   3345                3350                3355                3360
   Cys Cys Thr Ala Gly Cys Ala Ala Gly Gly Cys Ala Gly Gly Cys Ala
                   3365                3370                3375
   Thr Cys Thr Gly Gly Ala Gly Gly Gly Thr Gly Gly Ala Gly Thr Gly
               3380                3385                3390
   Cys Cys Thr Gly Ala Thr Cys Gly Gly Ala Gly Ala Gly Cys Ala Cys
           3395                3400                3405
   Cys Thr Gly Cys Ala Cys Gly Cys Ala Gly Gly Ala Ala Thr Gly Thr
       3410                    3415                    3420
   Cys Cys Ala Cys Cys Cys Thr Gly Thr Thr Thr Cys Thr Gly Gly Thr
   3425                    3430                    3435                    3440
   Gly Thr Ala Cys Thr Cys Thr Ala Ala Thr Ala Ala Gly Thr Gly Thr
                   3445                    3450                    3455
   Cys Ala Gly Ala Cys Ala Cys Cys Ala Cys Thr Gly Gly Gly Ala Ala
               3460                    3465                    3470
   Thr Gly Gly Cys Cys Ala Gly Cys Gly Gly Ala Cys Ala Cys Ala Thr
           3475                    3480                    3485
   Cys Ala Gly Gly Gly Ala Thr Thr Thr Cys Cys Ala Gly Ala Thr Cys
       3490                    3495                    3500
   Ala Cys Cys Gly Cys Cys Thr Cys Cys Gly Gly Ala Cys Ala Gly Thr
   3505                    3510                    3515                    3520
   Ala Cys Gly Gly Ala Cys Ala Gly Thr Gly Gly Gly Cys Ala Cys Cys
                   3525                    3530                    3535
   Thr Ala Ala Gly Cys Thr Gly Gly Cys Cys Cys Gly Cys Cys Thr Gly
               3540                    3545                    3550
   Cys Ala Cys Thr Ala Thr Thr Cys Thr Gly Gly Cys Ala Gly Cys Ala
           3555                    3560                    3565
   Thr Cys Ala Ala Cys Gly Cys Cys Thr Gly Gly Thr Cys Cys Ala Cys
       3570                    3575                    3580
   Cys Ala Ala Gly Gly Ala Gly Cys Cys Thr Thr Thr Cys Thr Cys Thr
   3585                    3590                    3595                    3600
   Thr Gly Gly Ala Thr Cys Ala Ala Gly Gly Thr Gly Gly Ala Cys Cys
                   3605                    3610                    3615
   Thr Gly Cys Thr Gly Gly Cys Cys Cys Cys Ala Ala Thr Gly Ala Thr
               3620                    3625                    3630
   Cys Ala Thr Cys Cys Ala Cys Gly Gly Cys Ala Thr Cys Ala Ala Gly
           3635                    3640                    3645
   Ala Cys Ala Cys Ala Gly Gly Gly Cys Gly Cys Cys Ala Gly Ala Cys
       3650                    3655                    3660
   Ala Gly Ala Ala Gly Thr Thr Cys Thr Cys Thr Ala Gly Cys Cys Thr
   3665                    3670                    3675                    3680
   Gly Thr Ala Cys Ala Thr Cys Ala Gly Cys Cys Ala Gly Thr Thr Thr
                   3685                    3690                    3695
   Ala Thr Cys Ala Thr Cys Ala Thr Gly Thr Ala Thr Thr Cys Cys Cys
               3700                    3705                    3710
   Thr Gly Gly Ala Thr Gly Gly Cys Ala Ala Gly Ala Ala Gly Thr Gly
           3715                    3720                    3725
   Gly Cys Ala Gly Ala Cys Cys Thr Ala Cys Ala Gly Ala Gly Gly Cys
       3730                    3735                    3740
   Ala Ala Thr Ala Gly Cys Ala Cys Cys Gly Gly Cys Ala Cys Ala Cys
   3745                3750                3755                3760
   Thr Gly Ala Thr Gly Gly Thr Gly Thr Thr Cys Thr Thr Thr Gly Gly
                   3765                3770                3775
   Cys Ala Ala Cys Gly Thr Gly Gly Ala Cys Thr Cys Cys Thr Cys Thr
               3780                3785                3790
   Gly Gly Cys Ala Thr Cys Ala Ala Gly Cys Ala Cys Ala Ala Cys Ala
           3795                3800                3805
   Thr Cys Thr Thr Cys Ala Ala Thr Cys Cys Thr Cys Cys Ala Ala Thr
       3810                3815                3820
   Cys Ala Thr Cys Gly Cys Cys Ala Gly Gly Thr Ala Cys Ala Thr Cys
   3825                3830                3835                3840
   Cys Gly Cys Cys Thr Gly Cys Ala Cys Cys Cys Ala Ala Cys Cys Cys
                   3845                3850                3855
   Ala Cys Thr Ala Thr Ala Gly Cys Ala Thr Cys Ala Gly Gly Thr Cys
               3860                3865                3870
   Cys Ala Cys Ala Cys Thr Gly Cys Gly Cys Ala Thr Gly Gly Ala Gly
           3875                3880                3885
   Cys Thr Gly Ala Thr Gly Gly Gly Cys Thr Gly Cys Gly Ala Thr Cys
       3890                3895                3900
   Thr Gly Ala Ala Cys Thr Cys Cys Thr Gly Thr Thr Cys Thr Ala Thr
   3905                3910                3915                3920
   Gly Cys Cys Cys Cys Thr Gly Gly Gly Cys Ala Thr Gly Gly Ala Gly
                   3925                3930                3935
   Thr Cys Cys Ala Ala Gly Gly Cys Cys Ala Thr Cys Thr Cys Thr Gly
               3940                3945                3950
   Ala Cys Gly Cys Cys Cys Ala Gly Ala Thr Cys Ala Cys Cys Gly Cys
           3955                3960                3965
   Cys Ala Gly Cys Thr Cys Cys Thr Ala Cys Thr Thr Cys Ala Cys Cys
       3970                3975                3980
   Ala Ala Thr Ala Thr Gly Thr Thr Thr Gly Cys Ala Ala Cys Ala Thr
   3985                3990                3995                4000
   Gly Gly Ala Gly Cys Cys Cys Cys Thr Cys Cys Ala Ala Gly Gly Cys
                   4005                4010                4015
   Cys Cys Gly Gly Cys Thr Gly Cys Ala Cys Cys Thr Gly Cys Ala Gly
               4020                4025                4030
   Gly Gly Cys Cys Gly Gly Thr Cys Cys Ala Ala Cys Gly Cys Ala Thr
           4035                4040                4045
   Gly Gly Cys Gly Gly Cys Cys Cys Cys Ala Gly Gly Thr Gly Ala Ala
       4050                4055                4060
   Cys Ala Ala Thr Cys Cys Thr Ala Ala Gly Gly Ala Gly Thr Gly Gly
   4065                4070                4075                4080
   Cys Thr Gly Cys Ala Gly Gly Thr Gly Gly Ala Thr Thr Thr Thr Cys
                   4085                4090                4095
   Ala Gly Ala Ala Ala Ala Cys Cys Ala Thr Gly Ala Ala Gly Gly Thr
               4100                4105                4110
   Gly Ala Cys Ala Gly Gly Cys Gly Thr Gly Ala Cys Cys Ala Cys Ala
           4115                4120                4125
   Cys Ala Gly Gly Gly Cys Gly Thr Gly Ala Ala Gly Ala Gly Cys Cys
       4130                4135                4140
   Thr Gly Cys Thr Gly Ala Cys Cys Thr Cys Cys Ala Thr Gly Thr Ala
   4145                4150                4155                4160
   Cys Gly Thr Gly Ala Ala Gly Gly Ala Gly Thr Thr Cys Cys Thr Gly
                   4165                4170                4175
   Ala Thr Cys Thr Cys Thr Ala Gly Cys Thr Cys Cys Cys Ala Gly Gly
               4180                4185                4190
   Ala Cys Gly Gly Cys Cys Ala Cys Cys Ala Gly Thr Gly Gly Ala Cys
           4195                4200                4205
   Ala Cys Thr Gly Thr Thr Cys Thr Thr Thr Cys Ala Gly Ala Ala Cys
       4210                4215                4220
   Gly Gly Cys Ala Ala Gly Gly Thr Gly Ala Ala Gly Gly Thr Gly Thr
   4225                4230                4235                4240
   Thr Cys Cys Ala Gly Gly Gly Cys Ala Ala Thr Cys Ala Gly Gly Ala
                   4245                4250                4255
   Thr Thr Cys Thr Thr Thr Thr Ala Cys Cys Cys Cys Thr Gly Thr Gly
               4260                4265                4270
   Gly Thr Gly Ala Ala Cys Ala Gly Cys Cys Thr Gly Gly Ala Cys Cys
           4275                4280                4285
   Cys Cys Cys Cys Thr Cys Thr Gly Cys Thr Gly Ala Cys Ala Cys Gly
       4290                4295                4300
   Gly Thr Ala Thr Cys Thr Gly Ala Gly Ala Ala Thr Cys Cys Ala Cys
   4305                4310                4315                4320
   Cys Cys Ala Cys Ala Gly Ala Gly Cys Thr Gly Gly Gly Thr Gly Cys
                   4325                4330                4335
   Ala Thr Cys Ala Gly Ala Thr Thr Gly Cys Cys Cys Thr Gly Cys Gly
               4340                4345                4350
   Ala Ala Thr Gly Gly Ala Ala Gly Thr Gly Cys Thr Gly Gly Gly Gly
           4355                4360                4365
   Thr Gly Thr Gly Ala Ala Gly Cys Thr Cys Ala Gly Gly Ala Cys Cys
       4370                4375                4380
   Thr Gly Thr Ala Thr Thr Gly Ala
   4385                4390

<210>    5

<211>    2366
<212>    DNA
<213>    Artificial Sequence

<220>
<223>    Fragment 1 for factor 8 mutant


<400>    5
atgcaaatag agctctccac ctgcttcttt ctgtgccttt tgcgattctg ctttagtgcc      60

accagaagat actacctggg tgcagtggaa ctgtcatggg actatatgca aagtgatctc     120

ggtgagctgc ctgtggacgc aagatttcct cctagagtgc caaaatcttt tccattcaac     180

acctcagtcg tgtacaaaaa gactctgttt gtagaattca cggatcacct tttcaacatc     240

gctaagccaa ggccaccctg gatgggtctg ctaggtccta ccatccaggc tgaggtttat     300

gatacagtgg tcattacact taagaacatg gcttcccatc ctgtcagtct tcatgctgtt     360

ggtgtatcct actggaaagc ttctgaggga gctgaatatg atgatcagac cagtcaaagg     420

gagaaagaag atgataaagt cttccctggt ggaagccata catatgtctg gcaggtcctg     480

aaagagaatg gtccaatggc ctctgaccca ctgtgcctta cctactcata tctttctcat     540

gtggacctgg taaaagactt gaattcaggc ctcattggag ccctactagt atgtagagaa     600

gggagtctgg ccaaggaaaa gacacagacc ttgcacaaat ttatactact ttttgctgta     660

tttgatgaag ggaaaagttg gcactcagaa acaaagaact ccttgatgca ggatagggat     720

gctgcatctg ctcgggcctg gcctaaaatg cacacagtca atggttatgt aaacaggtct     780

ctgccaggtc tgattggatg ccacaggaaa tcagtctatt ggcatgtgat tggaatgggc     840

accactcctg aagtgcactc aatattcctc gaaggtcaca catttcttgt gaggaaccat     900

cgccaggcgt ccttggaaat ctcgccaata actttcctta ctgctcaaac actcttgatg     960

gaccttggac agtttctact gttttgtcat atctcttccc accaacatga tggcatggaa    1020

gcttatgtca aagtagacag ctgtccagag gaaccccaac tacgaatgaa aaataatgaa    1080

gaagcggaag actatgatga tgatcttact gattctgaaa tggatgtggt caggtttgat    1140

gatgacaact ctccttcctt tatccaaatt cgctcagttg ccaagaagca tcctaaaact    1200

tgggtacatt acattgctgc tgaagaggag gactgggact atgctccctt agtcctcgcc    1260

cccgatgaca gaagttataa aagtcaatat ttgaacaatg gccctcagcg gattggtagg    1320

aagtacaaaa aagtccgatt tatggcatac acagatgaaa cctttaagac tcgtgaagct    1380

attcagcatg aatcaggaat cttgggacct ttactttatg gggaagttgg agacacactg    1440

ttgattatat ttaagaatca agcaagcaga ccatataaca tctaccctca cggaatcact    1500

gatgtccgtc ctttgtattc aaggagatta ccaaaaggtg taaaacattt gaaggatttt    1560

ccaattctgc caggagaaat attcaaatat aaatggacag tgactgtaga agatgggcca    1620

actaaatcag atcctcggtg cctgacccgc tattactcta gtttcgttaa tatggagaga 1680

gatctagctt caggactcat tggccctctc ctcatctgct acaaagaatc tgtagatcaa 1740

agaggaaacc agataatgtc agacaagagg aatgtcatcc tgttttctgt atttgatgag 1800

aaccgaagct ggtacctcac agagaatata caacgctttc tccccaatcc agctggagtg 1860

cagcttgagg atccagagtt ccaagcctcc aacatcatgc acagcatcaa tggctatgtt 1920

tttgatagtt tgcagttgtc agtttgtttg catgaggtgg catactggta cattctaagc 1980

attggagcac agactgactt cctttctgtc ttcttctctg gatatacctt caaacacaaa 2040

atggtctatg aagacacact caccctattc ccattctcag gagaaactgt cttcatgtcg 2100

atggaaaacc caggtctatg gattctgggg tgccacaact cagactttcg gaacagaggc 2160

atgaccgcct tactgaaggt ttctagttgt gacaagaaca ctggtgatta ttacgaggac 2220

agttatgaag atatttcagc atacttgctg agtaaaaaca atgccattga accaagaagc 2280

ttctcccaga attcaagaca ccctagcact aggcaaaagc aatttaatgc caccacaatt 2340

ccagaaaata ctactcttca gtcaga 2366

<210> 6
<211> 2060
<212> DNA
<213> Artificial Sequence

<220>
<223> Fragment 2 for factor 8 mutant

<400> 6
ccacaattcc agaaaatact actcttcagt cagatcaaga ggaaattgac tatgatgata 60

ccatatcagt tgaaatgaag aaggaagatt ttgacattta tgatgaggat gaaaatcaga 120

gcccccgcag ctttcaaaag aaaacacgac actattttat tgctgcagtg gagaggctct 180

gggattatgg gatgagtagc tccccacatg ttctaagaaa cagggctcag agtggcagtg 240

tccctcagtt caagaaagtt gttttccagg aatttactga tggctccttt actcagccct 300

tataccgtgg agaactaaat gaacatttgg gactcctggg gccatatata agagcagaag 360

ttgaagataa tatcatggta actttcagaa atcaggcctc tcgtccctat tccttctatt 420

ctagccttat ttcttatgag gaagatcaga ggcaaggagc agaacctaga aaaaactttg 480

tcaagcctaa tgaaaccaaa acttactttt ggaaagtgca acatcatatg gcacccacta 540

aagatgagtt tgactgcaaa gcctgggctt atttctctga tgttgacctg gaaaaagatg 600

tgcactcagg cctgattgga ccccttctgg tctgccacac taacacactg aaccctgctc 660

atgggagaca agtgacagta caggaatttg ctctgttttt caccatcttt gatgagacca 720

aaagctggta cttcactgaa aatatggaaa gaaactgcag ggctccctgc aatatccaga 780

tggaagatcc cacttttaaa gagaattatc gcttccatgc aatcaatggc tacataatgg 840

atacactacc tggcttagta atggctcagg atcaaaggat tcgatggtat ctgctcagca 900

tgggcagcaa tgaaaacatc cattctattc atttcagtgg acatgtgttc actgtacgaa 960

aaaaagagga gtataaaatg gcactgtaca atctctatcc aggtgttttt gagacagtgg 1020

aaatgttacc atccaaagct ggaatttggc gggtggaatg ccttattggc gagcatctac 1080

atgctgggat gagcacactt tttctggtgt acagcaataa gtgtcagact cccctgggaa 1140

tggcttctgg acacattaga gattttcaga ttacagcttc aggacaatat ggacagtggg 1200

ccccaaagct ggccagactt cattattccg gatcaatcaa tgcctggagc accaaggagc 1260

ccttttcttg gatcaaggtg gatctgttgg caccaatgat tattcacggc atcaagaccc 1320

agggtgcccg tcagaagttc tccagcctct acatctctca gtttatcatc atgtatagtc 1380

ttgatgggaa gaagtggcag acttatcgag gaaattccac tggaacctta atggtcttct 1440

ttggcaatgt ggattcatct gggataaaac acaatatttt taaccctcca attattgctc 1500

gatacatccg tttgcaccca actcattata gcattcgcag cactcttcgc atggagttga 1560

tgggctgtga tttaaatagt tgcagcatgc cattgggaat ggagagtaaa gcaatatcag 1620

atgcacagat tactgcttca tcctacttta ccaatatgtt tgccacctgg tctccttcaa 1680

aagctcgact tcacctccaa gggaggagta atgcctggag acctcaggtg aataatccaa 1740

aagagtggct gcaagtggac ttccagaaga caatgaaagt cacaggagta actactcagg 1800

gagtaaaatc tctgcttacc agcatgtatg tgaaggagtt cctcatctcc agcagtcaag 1860

atggccatca gtggactctc ttttttcaga atggcaaagt aaaggttttt cagggaaatc 1920

aagactcctt cacacctgtg gtgaactctc tagacccacc gttactgact cgctaccttc 1980

gaattcaccc ccagagttgg gtgcaccaga ttgccctgag gatggaggtt ctgggctgcg 2040

aggcacagga cctctactga 2060

<210> 7
<211> 2297
<212> DNA
<213> Artificial Sequence

<220>
<223> Fragment 3 for BDD-factor 8

<400> 7
atgcaaatag agctctccac ctgcttcttt ctgtgccttt tgcgattctg ctttagtgcc 60

accagaagat actacctggg tgcagtggaa ctgtcatggg actatatgca aagtgatctc 120

ggtgagctgc ctgtggacgc aagatttcct cctagagtgc caaaatcttt tccattcaac 180

acctcagtcg tgtacaaaaa gactctgttt gtagaattca cggatcacct tttcaacatc 240

gctaagccaa ggccaccctg gatgggtctg ctaggtccta ccatccaggc tgaggtttat 300

gatacagtgg tcattacact taagaacatg gcttcccatc ctgtcagtct tcatgctgtt 360

ggtgtatcct actggaaagc ttctgaggga gctgaatatg atgatcagac cagtcaaagg 420

gagaaagaag atgataaagt cttccctggt ggaagccata catatgtctg gcaggtcctg 480

aaagagaatg gtccaatggc ctctgaccca ctgtgcctta cctactcata tctttctcat 540

gtggacctgg taaaagactt gaattcaggc ctcattggag ccctactagt atgtagagaa 600

gggagtctgg ccaaggaaaa gacacagacc ttgcacaaat ttatactact ttttgctgta 660

tttgatgaag ggaaaagttg gcactcagaa acaaagaact ccttgatgca ggatagggat 720

gctgcatctg ctcgggcctg gcctaaaatg cacacagtca atggttatgt aaacaggtct 780

ctgccaggtc tgattggatg ccacaggaaa tcagtctatt ggcatgtgat tggaatgggc 840

accactcctg aagtgcactc aatattcctc gaaggtcaca catttcttgt gaggaaccat 900

cgccaggcgt ccttggaaat ctcgccaata actttcctta ctgctcaaac actcttgatg 960

gaccttggac agtttctact gttttgtcat atctcttccc accaacatga tggcatggaa 1020

gcttatgtca aagtagacag ctgtccagag gaaccccaac tacgaatgaa aaataatgaa 1080

gaagcggaag actatgatga tgatcttact gattctgaaa tggatgtggt caggtttgat 1140

gatgacaact ctccttcctt tatccaaatt cgctcagttg ccaagaagca tcctaaaact 1200

tgggtacatt acattgctgc tgaagaggag gactgggact atgctccctt agtcctcgcc 1260

cccgatgaca gaagttataa aagtcaatat ttgaacaatg gccctcagcg gattggtagg 1320

aagtacaaaa aagtccgatt tatggcatac acagatgaaa cctttaagac tcgtgaagct 1380

attcagcatg aatcaggaat cttgggacct ttactttatg gggaagttgg agacacactg 1440

ttgattatat ttaagaatca agcaagcaga ccatataaca tctaccctca cggaatcact 1500

gatgtccgtc ctttgtattc aaggagatta ccaaaaggtg taaaacattt gaaggatttt 1560

ccaattctgc caggagaaat attcaaatat aaatggacag tgactgtaga agatgggcca 1620

actaaatcag atcctcggtg cctgacccgc tattactcta gtttcgttaa tatggagaga 1680

gatctagctt caggactcat tggccctctc ctcatctgct acaaagaatc tgtagatcaa 1740

agaggaaacc agataatgtc agacaagagg aatgtcatcc tgttttctgt atttgatgag 1800

aaccgaagct ggtacctcac agagaatata caacgctttc tccccaatcc agctggagtg 1860

cagcttgagg atccagagtt ccaagcctcc aacatcatgc acagcatcaa tggctatgtt 1920

tttgatagtt tgcagttgtc agtttgtttg catgaggtgg catactggta cattctaagc 1980

attggagcac agactgactt cctttctgtc ttcttctctg gatatacctt caaacacaaa 2040

atggtctatg aagacacact caccctattc ccattctcag gagaaactgt cttcatgtcg 2100

atggaaaacc caggtctatg gattctgggg tgccacaact cagactttcg gaacagaggc 2160

atgaccgcct tactgaaggt ttctagttgt gacaagaaca ctggtgatta ttacgaggac 2220

agttatgaag atatttcagc atacttgctg agtaaaaaca atgccattga accaagagaa 2280

ataactcgta ctactct 2297

<210> 8
<211> 2064
<212> DNA
<213> Artificial Sequence

<220>
<223> Fragment 4 for BDD-factor 8

<400> 8
gaaccaagag aaataactcg tactactctt cagtcagatc aagaggaaat tgactatgat 60

gataccatat cagttgaaat gaagaaggaa gattttgaca tttatgatga ggatgaaaat 120

cagagccccc gcagctttca aaagaaaaca cgacactatt ttattgctgc agtggagagg 180

ctctgggatt atgggatgag tagctcccca catgttctaa gaaacagggc tcagagtggc 240

agtgtccctc agttcaagaa agttgttttc caggaattta ctgatggctc ctttactcag 300

cccttatacc gtggagaact aaatgaacat ttgggactcc tggggccata tataagagca 360

gaagttgaag ataatatcat ggtaactttc agaaatcagg cctctcgtcc ctattccttc 420

tattctagcc ttatttctta tgaggaagat cagaggcaag gagcagaacc tagaaaaaac 480

tttgtcaagc ctaatgaaac caaaacttac ttttggaaag tgcaacatca tatggcaccc 540

actaaagatg agtttgactg caaagcctgg gcttatttct ctgatgttga cctggaaaaa 600

gatgtgcact caggcctgat tggacccctt ctggtctgcc acactaacac actgaaccct 660

gctcatggga gacaagtgac agtacaggaa tttgctctgt ttttcaccat ctttgatgag 720

accaaaagct ggtacttcac tgaaaatatg gaaagaaact gcagggctcc ctgcaatatc 780

cagatggaag atcccacttt taaagagaat tatcgcttcc atgcaatcaa tggctacata 840

atggatacac tacctggctt agtaatggct caggatcaaa ggattcgatg gtatctgctc 900

agcatgggca gcaatgaaaa catccattct attcatttca gtggacatgt gttcactgta 960

cgaaaaaaag aggagtataa aatggcactg tacaatctct atccaggtgt ttttgagaca 1020

gtggaaatgt taccatccaa agctggaatt tggcgggtgg aatgccttat tggcgagcat 1080

ctacatgctg ggatgagcac actttttctg gtgtacagca ataagtgtca gactcccctg 1140

ggaatggctt ctggacacat tagagatttt cagattacag cttcaggaca atatggacag 1200

tgggccccaa agctggccag acttcattat tccggatcaa tcaatgcctg gagcaccaag 1260

gagccctttt cttggatcaa ggtggatctg ttggcaccaa tgattattca cggcatcaag 1320

acccagggtg cccgtcagaa gttctccagc ctctacatct ctcagtttat catcatgtat 1380

agtcttgatg ggaagaagtg gcagacttat cgaggaaatt ccactggaac cttaatggtc 1440

ttctttggca atgtggattc atctgggata aaacacaata tttttaaccc tccaattatt 1500

gctcgataca tccgtttgca cccaactcat tatagcattc gcagcactct tcgcatggag 1560

ttgatgggct gtgatttaaa tagttgcagc atgccattgg gaatggagag taaagcaata 1620

tcagatgcac agattactgc ttcatcctac tttaccaata tgtttgccac ctggtctcct 1680

tcaaaagctc gacttcacct ccaagggagg agtaatgcct ggagacctca ggtgaataat 1740

ccaaaagagt ggctgcaagt ggacttccag aagacaatga aagtcacagg agtaactact 1800

cagggagtaa aatctctgct taccagcatg tatgtgaagg agttcctcat ctccagcagt 1860

caagatggcc atcagtggac tctctttttt cagaatggca aagtaaaggt tttcagggga 1920

aatcaagact ccttcacacc tgtggtgaac tctctagacc caccgttact gactcgctac 1980

cttcgaattc acccccagag ttgggtgcac cagattgccc tgaggatgga ggttctgggc 2040

tgcgaggcac aggacctcta ctga 2064

<210> 9
<211> 2305
<212> DNA
<213> Artificial Sequence

<220>
<223> Fragment 5 for sc factor 8

<400> 9
atgcaaatag agctctccac ctgcttcttt ctgtgccttt tgcgattctg ctttagtgcc 60

accagaagat actacctggg tgcagtggaa ctgtcatggg actatatgca aagtgatctc 120

ggtgagctgc ctgtggacgc aagatttcct cctagagtgc caaaatcttt tccattcaac 180

acctcagtcg tgtacaaaaa gactctgttt gtagaattca cggatcacct tttcaacatc 240

gctaagccaa ggccaccctg gatgggtctg ctaggtccta ccatccaggc tgaggtttat 300

gatacagtgg tcattacact taagaacatg gcttcccatc ctgtcagtct tcatgctgtt 360

ggtgtatcct actggaaagc ttctgaggga gctgaatatg atgatcagac cagtcaaagg 420

gagaaagaag atgataaagt cttccctggt ggaagccata catatgtctg gcaggtcctg 480

aaagagaatg gtccaatggc ctctgaccca ctgtgcctta cctactcata tctttctcat 540

gtggacctgg taaaagactt gaattcaggc ctcattggag ccctactagt atgtagagaa 600

gggagtctgg ccaaggaaaa gacacagacc ttgcacaaat ttatactact ttttgctgta 660

tttgatgaag ggaaaagttg gcactcagaa acaaagaact ccttgatgca ggatagggat 720

gctgcatctg ctcgggcctg gcctaaaatg cacacagtca atggttatgt aaacaggtct 780

ctgccaggtc tgattggatg ccacaggaaa tcagtctatt ggcatgtgat tggaatgggc 840

accactcctg aagtgcactc aatattcctc gaaggtcaca catttcttgt gaggaaccat 900

cgccaggcgt ccttggaaat ctcgccaata actttcctta ctgctcaaac actcttgatg 960

gaccttggac agtttctact gttttgtcat atctcttccc accaacatga tggcatggaa 1020

gcttatgtca aagtagacag ctgtccagag gaaccccaac tacgaatgaa aaataatgaa 1080

gaagcggaag actatgatga tgatcttact gattctgaaa tggatgtggt caggtttgat 1140

gatgacaact ctccttcctt tatccaaatt cgctcagttg ccaagaagca tcctaaaact 1200

tgggtacatt acattgctgc tgaagaggag gactgggact atgctccctt agtcctcgcc 1260

cccgatgaca gaagttataa aagtcaatat ttgaacaatg gccctcagcg gattggtagg 1320

aagtacaaaa aagtccgatt tatggcatac acagatgaaa cctttaagac tcgtgaagct 1380

attcagcatg aatcaggaat cttgggacct ttactttatg gggaagttgg agacacactg 1440

ttgattatat ttaagaatca agcaagcaga ccatataaca tctaccctca cggaatcact 1500

gatgtccgtc ctttgtattc aaggagatta ccaaaaggtg taaaacattt gaaggatttt 1560

ccaattctgc caggagaaat attcaaatat aaatggacag tgactgtaga agatgggcca 1620

actaaatcag atcctcggtg cctgacccgc tattactcta gtttcgttaa tatggagaga 1680

gatctagctt caggactcat tggccctctc ctcatctgct acaaagaatc tgtagatcaa 1740

agaggaaacc agataatgtc agacaagagg aatgtcatcc tgttttctgt atttgatgag 1800

aaccgaagct ggtacctcac agagaatata caacgctttc tccccaatcc agctggagtg 1860

cagcttgagg atccagagtt ccaagcctcc aacatcatgc acagcatcaa tggctatgtt 1920

tttgatagtt tgcagttgtc agtttgtttg catgaggtgg catactggta cattctaagc 1980

attggagcac agactgactt cctttctgtc ttcttctctg gatatacctt caaacacaaa 2040

atggtctatg aagacacact caccctattc ccattctcag gagaaactgt cttcatgtcg 2100

atggaaaacc caggtctatg gattctgggg tgccacaact cagactttcg gaacagaggc 2160

atgaccgcct tactgaaggt ttctagttgt gacaagaaca ctggtgatta ttacgaggac 2220

agttatgaag atatttcagc atacttgctg agtaaaaaca atgccattga accaagaagc 2280

ttctcccaga atccaccagt cttga 2305

<210> 10
<211> 2095
<212> DNA
<213> Artificial Sequence

<220>
<223> Fragment 6 for sc factor 8

<400> 10
cttctcccag aatccaccag tcttgaaacg ccatcaacgg gaaataactc gtactactct 60

tcagtcagat caagaggaaa ttgactatga tgataccata tcagttgaaa tgaagaagga 120

agattttgac atttatgatg aggatgaaaa tcagagcccc cgcagctttc aaaagaaaac 180

acgacactat tttattgctg cagtggagag gctctgggat tatgggatga gtagctcccc 240

acatgttcta agaaacaggg ctcagagtgg cagtgtccct cagttcaaga aagttgtttt 300

ccaggaattt actgatggct cctttactca gcccttatac cgtggagaac taaatgaaca 360

tttgggactc ctggggccat atataagagc agaagttgaa gataatatca tggtaacttt 420

cagaaatcag gcctctcgtc cctattcctt ctattctagc cttatttctt atgaggaaga 480

tcagaggcaa ggagcagaac ctagaaaaaa ctttgtcaag cctaatgaaa ccaaaactta 540

cttttggaaa gtgcaacatc atatggcacc cactaaagat gagtttgact gcaaagcctg 600

ggcttatttc tctgatgttg acctggaaaa agatgtgcac tcaggcctga ttggacccct 660

tctggtctgc cacactaaca cactgaaccc tgctcatggg agacaagtga cagtacagga 720

atttgctctg tttttcacca tctttgatga gaccaaaagc tggtacttca ctgaaaatat 780

ggaaagaaac tgcagggctc cctgcaatat ccagatggaa gatcccactt ttaaagagaa 840

ttatcgcttc catgcaatca atggctacat aatggataca ctacctggct tagtaatggc 900

tcaggatcaa aggattcgat ggtatctgct cagcatgggc agcaatgaaa acatccattc 960

tattcatttc agtggacatg tgttcactgt acgaaaaaaa gaggagtata aaatggcact 1020

gtacaatctc tatccaggtg tttttgagac agtggaaatg ttaccatcca aagctggaat 1080

ttggcgggtg gaatgcctta ttggcgagca tctacatgct gggatgagca cactttttct 1140

ggtgtacagc aataagtgtc agactcccct gggaatggct tctggacaca ttagagattt 1200

tcagattaca gcttcaggac aatatggaca gtgggcccca aagctggcca gacttcatta 1260

ttccggatca atcaatgcct ggagcaccaa ggagcccttt tcttggatca aggtggatct 1320

gttggcacca atgattattc acggcatcaa gacccagggt gcccgtcaga agttctccag 1380

cctctacatc tctcagttta tcatcatgta tagtcttgat gggaagaagt ggcagactta 1440

tcgaggaaat tccactggaa ccttaatggt cttctttggc aatgtggatt catctgggat 1500

aaaacacaat atttttaacc ctccaattat tgctcgatac atccgtttgc acccaactca 1560

ttatagcatt cgcagcactc ttcgcatgga gttgatgggc tgtgatttaa atagttgcag 1620

catgccattg ggaatggaga gtaaagcaat atcagatgca cagattactg cttcatccta 1680

ctttaccaat atgtttgcca cctggtctcc ttcaaaagct cgacttcacc tccaagggag 1740

gagtaatgcc tggagacctc aggtgaataa tccaaaagag tggctgcaag tggacttcca 1800

gaagacaatg aaagtcacag gagtaactac tcagggagta aaatctctgc ttaccagcat 1860

gtatgtgaag gagttcctca tctccagcag tcaagatggc catcagtgga ctctcttttt 1920

tcagaatggc aaagtaaagg tttttcaggg aaatcaagac tccttcacac ctgtggtgaa 1980

ctctctagac ccaccgttac tgactcgcta ccttcgaatt cacccccaga gttgggtgca 2040

ccagattgcc ctgaggatgg aggttctggg ctgcgaggca caggacctct actga 2095

<210> 11
<211> 4374
<212> DNA
<213> Artificial Sequence

<220>
<223> sc factor 8

<400> 11
atgcaaatag agctctccac ctgcttcttt ctgtgccttt tgcgattctg ctttagtgcc 60

accagaagat actacctggg tgcagtggaa ctgtcatggg actatatgca aagtgatctc 120

ggtgagctgc ctgtggacgc aagatttcct cctagagtgc caaaatcttt tccattcaac 180

acctcagtcg tgtacaaaaa gactctgttt gtagaattca cggatcacct tttcaacatc 240

gctaagccaa ggccaccctg gatgggtctg ctaggtccta ccatccaggc tgaggtttat 300

gatacagtgg tcattacact taagaacatg gcttcccatc ctgtcagtct tcatgctgtt 360

ggtgtatcct actggaaagc ttctgaggga gctgaatatg atgatcagac cagtcaaagg 420

gagaaagaag atgataaagt cttccctggt ggaagccata catatgtctg gcaggtcctg 480

aaagagaatg gtccaatggc ctctgaccca ctgtgcctta cctactcata tctttctcat 540

gtggacctgg taaaagactt gaattcaggc ctcattggag ccctactagt atgtagagaa 600

gggagtctgg ccaaggaaaa gacacagacc ttgcacaaat ttatactact ttttgctgta 660

tttgatgaag ggaaaagttg gcactcagaa acaaagaact ccttgatgca ggatagggat 720

gctgcatctg ctcgggcctg gcctaaaatg cacacagtca atggttatgt aaacaggtct 780

ctgccaggtc tgattggatg ccacaggaaa tcagtctatt ggcatgtgat tggaatgggc 840

accactcctg aagtgcactc aatattcctc gaaggtcaca catttcttgt gaggaaccat 900

cgccaggcgt ccttggaaat ctcgccaata actttcctta ctgctcaaac actcttgatg 960

gaccttggac agtttctact gttttgtcat atctcttccc accaacatga tggcatggaa 1020

gcttatgtca aagtagacag ctgtccagag gaaccccaac tacgaatgaa aaataatgaa 1080

gaagcggaag actatgatga tgatcttact gattctgaaa tggatgtggt caggtttgat 1140

gatgacaact ctccttcctt tatccaaatt cgctcagttg ccaagaagca tcctaaaact 1200

tgggtacatt acattgctgc tgaagaggag gactgggact atgctccctt agtcctcgcc 1260

cccgatgaca gaagttataa aagtcaatat ttgaacaatg gccctcagcg gattggtagg 1320

aagtacaaaa aagtccgatt tatggcatac acagatgaaa cctttaagac tcgtgaagct 1380

attcagcatg aatcaggaat cttgggacct ttactttatg gggaagttgg agacacactg 1440

ttgattatat ttaagaatca agcaagcaga ccatataaca tctaccctca cggaatcact 1500

gatgtccgtc ctttgtattc aaggagatta ccaaaaggtg taaaacattt gaaggatttt 1560

ccaattctgc caggagaaat attcaaatat aaatggacag tgactgtaga agatgggcca 1620

actaaatcag atcctcggtg cctgacccgc tattactcta gtttcgttaa tatggagaga 1680

gatctagctt caggactcat tggccctctc ctcatctgct acaaagaatc tgtagatcaa 1740

agaggaaacc agataatgtc agacaagagg aatgtcatcc tgttttctgt atttgatgag 1800

aaccgaagct ggtacctcac agagaatata caacgctttc tccccaatcc agctggagtg 1860

cagcttgagg atccagagtt ccaagcctcc aacatcatgc acagcatcaa tggctatgtt 1920

tttgatagtt tgcagttgtc agtttgtttg catgaggtgg catactggta cattctaagc 1980

attggagcac agactgactt cctttctgtc ttcttctctg gatatacctt caaacacaaa 2040

atggtctatg aagacacact caccctattc ccattctcag gagaaactgt cttcatgtcg 2100

atggaaaacc caggtctatg gattctgggg tgccacaact cagactttcg gaacagaggc 2160

atgaccgcct tactgaaggt ttctagttgt gacaagaaca ctggtgatta ttacgaggac 2220

agttatgaag atatttcagc atacttgctg agtaaaaaca atgccattga accaagaagc 2280

ttctcccaga atccaccagt cttgaaacgc catcaacggg aaataactcg tactactctt 2340

cagtcagatc aagaggaaat tgactatgat gataccatat cagttgaaat gaagaaggaa 2400

gattttgaca tttatgatga ggatgaaaat cagagccccc gcagctttca aaagaaaaca 2460

cgacactatt ttattgctgc agtggagagg ctctgggatt atgggatgag tagctcccca 2520

catgttctaa gaaacagggc tcagagtggc agtgtccctc agttcaagaa agttgttttc 2580

caggaattta ctgatggctc ctttactcag cccttatacc gtggagaact aaatgaacat 2640

ttgggactcc tggggccata tataagagca gaagttgaag ataatatcat ggtaactttc 2700

agaaatcagg cctctcgtcc ctattccttc tattctagcc ttatttctta tgaggaagat 2760

cagaggcaag gagcagaacc tagaaaaaac tttgtcaagc ctaatgaaac caaaacttac 2820

ttttggaaag tgcaacatca tatggcaccc actaaagatg agtttgactg caaagcctgg 2880

gcttatttct ctgatgttga cctggaaaaa gatgtgcact caggcctgat tggacccctt 2940

ctggtctgcc acactaacac actgaaccct gctcatggga gacaagtgac agtacaggaa 3000

tttgctctgt ttttcaccat ctttgatgag accaaaagct ggtacttcac tgaaaatatg 3060

gaaagaaact gcagggctcc ctgcaatatc cagatggaag atcccacttt taaagagaat 3120

tatcgcttcc atgcaatcaa tggctacata atggatacac tacctggctt agtaatggct 3180

caggatcaaa ggattcgatg gtatctgctc agcatgggca gcaatgaaaa catccattct 3240

attcatttca gtggacatgt gttcactgta cgaaaaaaag aggagtataa aatggcactg 3300

tacaatctct atccaggtgt ttttgagaca gtggaaatgt taccatccaa agctggaatt 3360

tggcgggtgg aatgccttat tggcgagcat ctacatgctg ggatgagcac actttttctg 3420

gtgtacagca ataagtgtca gactcccctg ggaatggctt ctggacacat tagagatttt 3480

cagattacag cttcaggaca atatggacag tgggccccaa agctggccag acttcattat 3540

tccggatcaa tcaatgcctg gagcaccaag gagccctttt cttggatcaa ggtggatctg 3600

ttggcaccaa tgattattca cggcatcaag acccagggtg cccgtcagaa gttctccagc 3660

ctctacatct ctcagtttat catcatgtat agtcttgatg ggaagaagtg gcagacttat 3720

cgaggaaatt ccactggaac cttaatggtc ttctttggca atgtggattc atctgggata 3780

aaacacaata tttttaaccc tccaattatt gctcgataca tccgtttgca cccaactcat 3840

tatagcattc gcagcactct tcgcatggag ttgatgggct gtgatttaaa tagttgcagc 3900

atgccattgg gaatggagag taaagcaata tcagatgcac agattactgc ttcatcctac 3960

tttaccaata tgtttgccac ctggtctcct tcaaaagctc gacttcacct ccaagggagg 4020

agtaatgcct ggagacctca ggtgaataat ccaaaagagt ggctgcaagt ggacttccag 4080

aagacaatga aagtcacagg agtaactact cagggagtaa aatctctgct taccagcatg 4140

tatgtgaagg agttcctcat ctccagcagt caagatggcc atcagtggac tctctttttt 4200

cagaatggca aagtaaaggt ttttcaggga aatcaagact ccttcacacc tgtggtgaac 4260

tctctagacc caccgttact gactcgctac cttcgaattc acccccagag ttgggtgcac 4320

cagattgccc tgaggatgga ggttctgggc tgcgaggcac aggacctcta ctga 4374

<210> 12
<211> 5431
<212> DNA
<213> Artificial Sequence

<220>
<223> pCDNA3.1

<400> 12
gacggatcgg gagatctccc gatcccctat ggtgcactct cagtacaatc tgctctgatg 60

ccgcatagtt aagccagtat ctgctccctg cttgtgtgtt ggaggtcgct gagtagtgcg 120

cgagcaaaat ttaagctaca acaaggcaag gcttgaccga caattgcatg aagaatctgc 180

ttagggttag gcgttttgcg ctgcttcgcg atgtacgggc cagatatacg cgttgacatt 240

gattattgac tagttattaa tagtaatcaa ttacggggtc attagttcat agcccatata 300

tggagttccg cgttacataa cttacggtaa atggcccgcc tggctgaccg cccaacgacc 360

cccgcccatt gacgtcaata atgacgtatg ttcccatagt aacgccaata gggactttcc 420

attgacgtca atgggtggag tatttacggt aaactgccca cttggcagta catcaagtgt 480

atcatatgcc aagtacgccc cctattgacg tcaatgacgg taaatggccc gcctggcatt 540

atgcccagta catgacctta tgggactttc ctacttggca gtacatctac gtattagtca 600

tcgctattac catggtgatg cggttttggc agtacatcaa tgggcgtgga tagcggtttg 660

actcacgggg atttccaagt ctccacccca ttgacgtcaa tgggagtttg ttttggcacc 720

aaaatcaacg ggactttcca aaatgtcgta acaactccgc cccattgacg caaatgggcg 780

gtaggcgtgt acggtgggag gtctatataa gcagagctct ctggctaact agagaaccca 840

ctgcttactg gcttatcgaa attaatacga ctcactatag ggagacccaa gctggctagc 900

gtttaaactt aagcttggta ccgagctcgg atccactagt ccagtgtggt ggaattctgc 960

agatatccag cacagtggcg gccgctcgag tctagagggc ccgtttaaac ccgctgatca 1020

gcctcgactg tgccttctag ttgccagcca tctgttgttt gcccctcccc cgtgccttcc 1080

ttgaccctgg aaggtgccac tcccactgtc ctttcctaat aaaatgagga aattgcatcg 1140

cattgtctga gtaggtgtca ttctattctg gggggtgggg tggggcagga cagcaagggg 1200

gaggattggg aagacaatag caggcatgct ggggatgcgg tgggctctat ggcttctgag 1260

gcggaaagaa ccagctgggg ctctaggggg tatccccacg cgccctgtag cggcgcatta 1320

agcgcggcgg gtgtggtggt tacgcgcagc gtgaccgcta cacttgccag cgccctagcg 1380

cccgctcctt tcgctttctt cccttccttt ctcgccacgt tcgccggctt tccccgtcaa 1440

gctctaaatc gggggctccc tttagggttc cgatttagtg ctttacggca cctcgacccc 1500

aaaaaacttg attagggtga tggttcacgt agtgggccat cgccctgata gacggttttt 1560

cgccctttga cgttggagtc cacgttcttt aatagtggac tcttgttcca aactggaaca 1620

acactcaacc ctatctcggt ctattctttt gatttataag ggattttgcc gatttcggcc 1680

tattggttaa aaaatgagct gatttaacaa aaatttaacg cgaattaatt ctgtggaatg 1740

tgtgtcagtt agggtgtgga aagtccccag gctccccagc aggcagaagt atgcaaagca 1800

tgcatctcaa ttagtcagca accaggtgtg gaaagtcccc aggctcccca gcaggcagaa 1860

gtatgcaaag catgcatctc aattagtcag caaccatagt cccgccccta actccgccca 1920

tcccgcccct aactccgccc agttccgccc attctccgcc ccatggctga ctaatttttt 1980

ttatttatgc agaggccgag gccgcctctg cctctgagct attccagaag tagtgaggag 2040

gcttttttgg aggcctaggc ttttgcaaaa agctcccggg agcttgtata tccattttcg 2100

gatctgatca agagacagga tgaggatcgt ttcgcatgat tgaacaagat ggattgcacg 2160

caggttctcc ggccgcttgg gtggagaggc tattcggcta tgactgggca caacagacaa 2220

tcggctgctc tgatgccgcc gtgttccggc tgtcagcgca ggggcgcccg gttctttttg 2280

tcaagaccga cctgtccggt gccctgaatg aactgcagga cgaggcagcg cggctatcgt 2340

ggctggccac gacgggcgtt ccttgcgcag ctgtgctcga cgttgtcact gaagcgggaa 2400

gggactggct gctattgggc gaagtgccgg ggcaggatct cctgtcatct caccttgctc 2460

ctgccgagaa agtatccatc atggctgatg caatgcggcg gctgcatacg cttgatccgg 2520

ctacctgccc attcgaccac caagcgaaac atcgcatcga gcgagcacgt actcggatgg 2580

aagccggtct tgtcgatcag gatgatctgg acgaagagca tcaggggctc gcgccagccg 2640

aactgttcgc caggctcaag gcgcgcatgc ccgacggcga ggatctcgtc gtgacccatg 2700

gcgatgcctg cttgccgaat atcatggtgg aaaatggccg cttttctgga ttcatcgact 2760

gtggccggct gggtgtggcg gaccgctatc aggacatagc gttggctacc cgtgatattg 2820

ctgaagagct tggcggcgaa tgggctgacc gcttcctcgt gctttacggt atcgccgctc 2880

ccgattcgca gcgcatcgcc ttctatcgcc ttcttgacga gttcttctga gcgggactct 2940

ggggttcgaa atgaccgacc aagcgacgcc caacctgcca tcacgagatt tcgattccac 3000

cgccgccttc tatgaaaggt tgggcttcgg aatcgttttc cgggacgccg gctggatgat 3060

cctccagcgc ggggatctca tgctggagtt cttcgcccac cccaacttgt ttattgcagc 3120

ttataatggt tacaaataaa gcaatagcat cacaaatttc acaaataaag catttttttc 3180

actgcattct agttgtggtt tgtccaaact catcaatgta tcttatcatg tctgtatacc 3240

gtcgacctct agctagagct tggcgtaatc atggtcatag ctgtttcctg tgtgaaattg 3300

ttatccgctc acaattccac acaacatacg agccggaagc ataaagtgta aagcctgggg 3360

tgcctaatga gtgagctaac tcacattaat tgcgttgcgc tcactgcccg ctttccagtc 3420

gggaaacctg tcgtgccagc tgcattaatg aatcggccaa cgcgcgggga gaggcggttt 3480

gcgtattggg cgctcttccg cttcctcgct cactgactcg ctgcgctcgg tcgttcggct 3540

gcggcgagcg gtatcagctc actcaaaggc ggtaatacgg ttatccacag aatcagggga 3600

taacgcagga aagaacatgt gagcaaaagg ccagcaaaag gccaggaacc gtaaaaaggc 3660

cgcgttgctg gcgtttttcc ataggctccg cccccctgac gagcatcaca aaaatcgacg 3720

ctcaagtcag aggtggcgaa acccgacagg actataaaga taccaggcgt ttccccctgg 3780

aagctccctc gtgcgctctc ctgttccgac cctgccgctt accggatacc tgtccgcctt 3840

tctcccttcg ggaagcgtgg cgctttctca tagctcacgc tgtaggtatc tcagttcggt 3900

gtaggtcgtt cgctccaagc tgggctgtgt gcacgaaccc cccgttcagc ccgaccgctg 3960

cgccttatcc ggtaactatc gtcttgagtc caacccggta agacacgact tatcgccact 4020

ggcagcagcc actggtaaca ggattagcag agcgaggtat gtaggcggtg ctacagagtt 4080

cttgaagtgg tggcctaact acggctacac tagaagaaca gtatttggta tctgcgctct 4140

gctgaagcca gttaccttcg gaaaaagagt tggtagctct tgatccggca aacaaaccac 4200

cgctggtagc ggtggttttt ttgtttgcaa gcagcagatt acgcgcagaa aaaaaggatc 4260

tcaagaagat cctttgatct tttctacggg gtctgacgct cagtggaacg aaaactcacg 4320

ttaagggatt ttggtcatga gattatcaaa aaggatcttc acctagatcc ttttaaatta 4380

aaaatgaagt tttaaatcaa tctaaagtat atatgagtaa acttggtctg acagttacca 4440

atgcttaatc agtgaggcac ctatctcagc gatctgtcta tttcgttcat ccatagttgc 4500

ctgactcccc gtcgtgtaga taactacgat acgggagggc ttaccatctg gccccagtgc 4560

tgcaatgata ccgcgagacc cacgctcacc ggctccagat ttatcagcaa taaaccagcc 4620

agccggaagg gccgagcgca gaagtggtcc tgcaacttta tccgcctcca tccagtctat 4680

taattgttgc cgggaagcta gagtaagtag ttcgccagtt aatagtttgc gcaacgttgt 4740

tgccattgct acaggcatcg tggtgtcacg ctcgtcgttt ggtatggctt cattcagctc 4800

cggttcccaa cgatcaaggc gagttacatg atcccccatg ttgtgcaaaa aagcggttag 4860

ctccttcggt cctccgatcg ttgtcagaag taagttggcc gcagtgttat cactcatggt 4920

tatggcagca ctgcataatt ctcttactgt catgccatcc gtaagatgct tttctgtgac 4980

tggtgagtac tcaaccaagt cattctgaga atagtgtatg cggcgaccga gttgctcttg 5040

cccggcgtca atacgggata ataccgcgcc acatagcaga actttaaaag tgctcatcat 5100

tggaaaacgt tcttcggggc gaaaactctc aaggatctta ccgctgttga gatccagttc 5160

gatgtaaccc actcgtgcac ccaactgatc ttcagcatct tttactttca ccagcgtttc 5220

tgggtgagca aaaacaggaa ggcaaaatgc cgcaaaaaag ggaataaggg cgacacggaa 5280

atgttgaata ctcatactct tcctttttca atattattga agcatttatc agggttattg 5340

tctcatgagc ggatacatat ttgaatgtat ttagaaaaat aaacaaatag gggttccgcg 5400

cacatttccc cgaaaagtgc cacctgacgt c 5431

<210> 13
<211> 4374
<212> DNA
<213> Artificial Sequence

<220>
<223> pGP

<400> 13
atgcaaatag agctctccac ctgcttcttt ctgtgccttt tgcgattctg ctttagtgcc 60

accagaagat actacctggg tgcagtggaa ctgtcatggg actatatgca aagtgatctc 120

ggtgagctgc ctgtggacgc aagatttcct cctagagtgc caaaatcttt tccattcaac 180

acctcagtcg tgtacaaaaa gactctgttt gtagaattca cggatcacct tttcaacatc 240

gctaagccaa ggccaccctg gatgggtctg ctaggtccta ccatccaggc tgaggtttat 300

gatacagtgg tcattacact taagaacatg gcttcccatc ctgtcagtct tcatgctgtt 360

ggtgtatcct actggaaagc ttctgaggga gctgaatatg atgatcagac cagtcaaagg 420

gagaaagaag atgataaagt cttccctggt ggaagccata catatgtctg gcaggtcctg 480

aaagagaatg gtccaatggc ctctgaccca ctgtgcctta cctactcata tctttctcat 540

gtggacctgg taaaagactt gaattcaggc ctcattggag ccctactagt atgtagagaa 600

gggagtctgg ccaaggaaaa gacacagacc ttgcacaaat ttatactact ttttgctgta 660

tttgatgaag ggaaaagttg gcactcagaa acaaagaact ccttgatgca ggatagggat 720

gctgcatctg ctcgggcctg gcctaaaatg cacacagtca atggttatgt aaacaggtct 780

ctgccaggtc tgattggatg ccacaggaaa tcagtctatt ggcatgtgat tggaatgggc 840

accactcctg aagtgcactc aatattcctc gaaggtcaca catttcttgt gaggaaccat 900

cgccaggcgt ccttggaaat ctcgccaata actttcctta ctgctcaaac actcttgatg 960

gaccttggac agtttctact gttttgtcat atctcttccc accaacatga tggcatggaa 1020

gcttatgtca aagtagacag ctgtccagag gaaccccaac tacgaatgaa aaataatgaa 1080

gaagcggaag actatgatga tgatcttact gattctgaaa tggatgtggt caggtttgat 1140

gatgacaact ctccttcctt tatccaaatt cgctcagttg ccaagaagca tcctaaaact 1200

tgggtacatt acattgctgc tgaagaggag gactgggact atgctccctt agtcctcgcc 1260

cccgatgaca gaagttataa aagtcaatat ttgaacaatg gccctcagcg gattggtagg 1320

aagtacaaaa aagtccgatt tatggcatac acagatgaaa cctttaagac tcgtgaagct 1380

attcagcatg aatcaggaat cttgggacct ttactttatg gggaagttgg agacacactg 1440

ttgattatat ttaagaatca agcaagcaga ccatataaca tctaccctca cggaatcact 1500

gatgtccgtc ctttgtattc aaggagatta ccaaaaggtg taaaacattt gaaggatttt 1560

ccaattctgc caggagaaat attcaaatat aaatggacag tgactgtaga agatgggcca 1620

actaaatcag atcctcggtg cctgacccgc tattactcta gtttcgttaa tatggagaga 1680

gatctagctt caggactcat tggccctctc ctcatctgct acaaagaatc tgtagatcaa 1740

agaggaaacc agataatgtc agacaagagg aatgtcatcc tgttttctgt atttgatgag 1800

aaccgaagct ggtacctcac agagaatata caacgctttc tccccaatcc agctggagtg 1860

cagcttgagg atccagagtt ccaagcctcc aacatcatgc acagcatcaa tggctatgtt 1920

tttgatagtt tgcagttgtc agtttgtttg catgaggtgg catactggta cattctaagc 1980

attggagcac agactgactt cctttctgtc ttcttctctg gatatacctt caaacacaaa 2040

atggtctatg aagacacact caccctattc ccattctcag gagaaactgt cttcatgtcg 2100

atggaaaacc caggtctatg gattctgggg tgccacaact cagactttcg gaacagaggc 2160

atgaccgcct tactgaaggt ttctagttgt gacaagaaca ctggtgatta ttacgaggac 2220

agttatgaag atatttcagc atacttgctg agtaaaaaca atgccattga accaagaagc 2280

ttctcccaga atccaccagt cttgaaacgc catcaacggg aaataactcg tactactctt 2340

cagtcagatc aagaggaaat tgactatgat gataccatat cagttgaaat gaagaaggaa 2400

gattttgaca tttatgatga ggatgaaaat cagagccccc gcagctttca aaagaaaaca 2460

cgacactatt ttattgctgc agtggagagg ctctgggatt atgggatgag tagctcccca 2520

catgttctaa gaaacagggc tcagagtggc agtgtccctc agttcaagaa agttgttttc 2580

caggaattta ctgatggctc ctttactcag cccttatacc gtggagaact aaatgaacat 2640

ttgggactcc tggggccata tataagagca gaagttgaag ataatatcat ggtaactttc 2700

agaaatcagg cctctcgtcc ctattccttc tattctagcc ttatttctta tgaggaagat 2760

cagaggcaag gagcagaacc tagaaaaaac tttgtcaagc ctaatgaaac caaaacttac 2820

ttttggaaag tgcaacatca tatggcaccc actaaagatg agtttgactg caaagcctgg 2880

gcttatttct ctgatgttga cctggaaaaa gatgtgcact caggcctgat tggacccctt 2940

ctggtctgcc acactaacac actgaaccct gctcatggga gacaagtgac agtacaggaa 3000

tttgctctgt ttttcaccat ctttgatgag accaaaagct ggtacttcac tgaaaatatg 3060

gaaagaaact gcagggctcc ctgcaatatc cagatggaag atcccacttt taaagagaat 3120

tatcgcttcc atgcaatcaa tggctacata atggatacac tacctggctt agtaatggct 3180

caggatcaaa ggattcgatg gtatctgctc agcatgggca gcaatgaaaa catccattct 3240

attcatttca gtggacatgt gttcactgta cgaaaaaaag aggagtataa aatggcactg 3300

tacaatctct atccaggtgt ttttgagaca gtggaaatgt taccatccaa agctggaatt 3360

tggcgggtgg aatgccttat tggcgagcat ctacatgctg ggatgagcac actttttctg 3420

gtgtacagca ataagtgtca gactcccctg ggaatggctt ctggacacat tagagatttt 3480

cagattacag cttcaggaca atatggacag tgggccccaa agctggccag acttcattat 3540

tccggatcaa tcaatgcctg gagcaccaag gagccctttt cttggatcaa ggtggatctg 3600

ttggcaccaa tgattattca cggcatcaag acccagggtg cccgtcagaa gttctccagc 3660

ctctacatct ctcagtttat catcatgtat agtcttgatg ggaagaagtg gcagacttat 3720

cgaggaaatt ccactggaac cttaatggtc ttctttggca atgtggattc atctgggata 3780

aaacacaata tttttaaccc tccaattatt gctcgataca tccgtttgca cccaactcat 3840

tatagcattc gcagcactct tcgcatggag ttgatgggct gtgatttaaa tagttgcagc 3900

atgccattgg gaatggagag taaagcaata tcagatgcac agattactgc ttcatcctac 3960

tttaccaata tgtttgccac ctggtctcct tcaaaagctc gacttcacct ccaagggagg 4020

agtaatgcct ggagacctca ggtgaataat ccaaaagagt ggctgcaagt ggacttccag 4080

aagacaatga aagtcacagg agtaactact cagggagtaa aatctctgct taccagcatg 4140

tatgtgaagg agttcctcat ctccagcagt caagatggcc atcagtggac tctctttttt 4200

cagaatggca aagtaaaggt ttttcaggga aatcaagact ccttcacacc tgtggtgaac 4260

tctctagacc caccgttact gactcgctac cttcgaattc acccccagag ttgggtgcac 4320

cagattgccc tgaggatgga ggttctgggc tgcgaggcac aggacctcta ctga 4374

<210> 14
<211> 5633
<212> DNA
<213> Artificial Sequence

<220>
<223> pEF vector

<400> 14
ccaattcaag cttcgtgagg ctccggtgcc cgtcagtggg cagagcgcac atcgcccaca 60

gtccccgaga agttgggggg aggggtcggc aattgaaccg gtgcctagag aaggtggcgc 120

ggggtaaact gggaaagtga tgtcgtgtac tggctccgcc tttttcccga gggtggggga 180

gaaccgtata taagtgcagt agtcgccgtg aacgttcttt ttcgcaacgg gtttgccgcc 240

agaacacagg taagtgccgt gtgtggttcc cgcgggcctg gcctctttac gggttatggc 300

ccttgcgtgc cttgaattac ttccacctgg ctccagtacg tgattcttga tcccgagctg 360

gagccagggg cgggccttgc gctttaggag ccccttcgcc tcgtgcttga gttgaggcct 420

ggcctgggcg ctggggccgc cgcgtgcgaa tctggtggca ccttcgcgcc tgtctcgctg 480

ctttcgataa gtctctagcc atttaaaatt tttgatgacc tgctgcgacg ctttttttct 540

ggcaagatag tcttgtaaat gcgggccagg atctgcacac tggtatttcg gtttttgggc 600

ccgcggccgg cgacggggcc cgtgcgtccc agcgcacatg ttcggcgagg cggggcctgc 660

gagcgcggcc accgagaatc ggacgggggt agtctcaagc tggccggcct gctctggtgc 720

ctggcctcgc gccgccgtgt atcgccccgc cctgggcggc aaggctggcc cggtcggcac 780

cagttgcgtg agcggaaaga tggccgcttc ccggccctgc tccagggggc tcaaaatgga 840

ggacgcggcg ctcgggagag cgggcgggtg agtcacccac acaaaggaaa agggcctttc 900

cgtcctcagc cgtcgcttca tgtgactcca cggagtaccg ggcgccgtcc aggcacctcg 960

attagttctg gagcttttgg agtacgtcgt ctttaggttg gggggagggg ttttatgcga 1020

tggagtttcc ccacactgag tgggtggaga ctgaagttag gccagcttgg cacttgatgt 1080

aattctcctt ggaatttggc ctttttgagt ttggatcttg gttcattctc aagcctcaga 1140

cagtggttca aagttttttt cttccatttc aggtgtcgtg aacacgggct gcagaagttg 1200

gtcgtgaggc actgggcagg taagtatcaa ggttacaaga caggtttaag gagaccaata 1260

gaaactgggc ttgtcgagac agagaagact cttgcgtttc tgataggcac ctattggtct 1320

tactgacatc cactttgcct ttctctccac aggtgtccac tcccagttca attacagctc 1380

ttaaggctag agtacttaat acgactcact ataggctagc ctcgagatcc tagagtcgac 1440

ccgggcggcc gctctagccc aattccgccc ctctccctcc ccccccccta acgttactgg 1500

ccgaagccgc ttggaataag gccggtgtgc gtttgtctat atgttatttt ccaccatatt 1560

gccgtctttt ggcaatgtga gggcccggaa acctggccct gtcttcttga cgagcattcc 1620

taggggtctt tcccctctcg ccaaaggaat gcaaggtctg ttgaatgtcg tgaaggaagc 1680

agttcctctg gaagcttctt gaagacaaac aacgtctgta gcgacccttt gcaggcagcg 1740

gaacccccca cctggcgaca ggtgcctctg cggccaaaag ccacgtgtat aagatacacc 1800

tgcaaaggcg gcacaacccc agtgccacgt tgtgagttgg atagttgtgg aaagagtcaa 1860

atggctctcc tcaagcgtat tcaacaaggg gctgaaggat gcccagaagg taccccattg 1920

tatgggatct gatctggggc ctcggtgcac atgctttaca tgtgtttagt cgaggttaaa 1980

aaacgtctag gccccccgaa ccacggggac gtggttttcc tttgaaaaac acgatgataa 2040

tatgggcatt gaacaagatg gattgcacgc aggttctccg gccgcttggg tggagaggct 2100

attcggctat gactgggcac aacagacaat cggctgctct gatgccgccg tgttccggct 2160

gtcagcgcag gggcgcccgg ttctttttgt caagaccgac ctgtccggtg ccctgaatga 2220

actgcaggac gaggcagcgc ggctatcgtg gctggccacg acgggcgttc cttgcgcagc 2280

tgtgctcgac gttgtcactg aagcgggaag ggactggctg ctattgggcg aagtgccggg 2340

gcaggatctc ctgtcatctc accttgctcc tgccgagaaa gtatccatca tggctgatgc 2400

aatgcggcgg ctgcatacgc ttgatccggc tacctgccca ttcgaccacc aagcgaaaca 2460

tcgcatcgag cgagcacgta ctcggatgga agccggtctt gtcgatcagg atgatctgga 2520

cgaagagcat caggggctcg cgccagccga actgttcgcc aggctcaagg cgcgcatgcc 2580

cgacggcgag gatctcgtcg tgacccatgg cgatgcctgc ttgccgaata tcatggtgga 2640

aaatggccgc ttttctggat tcatcgactg tggccggctg ggtgtggcgg accgctatca 2700

ggacatagcg ttggctaccc gtgatattgc tgaagagctt ggcggcgaat gggctgaccg 2760

cttcctcgtg ctttacggta tcgccgctcc cgattcgcag cgcatcgcct tctatcgcct 2820

tcttgacgag ttcttctgag cgggactctg gggttcgaaa tgaccgacca agcgacgccc 2880

aacctgccat cacgatggcc gcaataaaat atctttattt tcattacatc tgtgtgttgg 2940

ttttttgtgt gaatcgatag cgataaggat ccgggctggc gtaatagcga agaggcccgc 3000

accgatcgcc cttcccaaca gttgcgcagc ctgaatggcg aatggacgcg ccctgtagcg 3060

gcgcattaag cgcggcgggt gtggtggtta cgcgcagcgt gaccgctaca cttgccagcg 3120

ccctagcgcc cgctcctttc gctttcttcc cttcctttct cgccacgttc gccggctttc 3180

cccgtcaagc tctaaatcgg gggctccctt tagggttccg atttagagct ttacggcacc 3240

tcgaccgcaa aaaacttgat ttgggtgatg gttcacgtag tgggccatcg ccctgataga 3300

cggtttttcg ccctttgacg ttggagtcca cgttctttaa tagtggactc ttgttccaaa 3360

ctggaacaac actcaaccct atctcggtct attcttttga tttataaggg attttgccga 3420

tttcggccta ttggttaaaa aatgagctga tttaacaaat atttaacgcg aattttaaca 3480

aaatattaac gtttacaatt tcgcctgatg cggtattttc tccttacgca tctgtgcggt 3540

atttcacacc gcatatggtg cactctcagt acaatctgct ctgatgccgc atagttaagc 3600

cagccccgac acccgccaac acccgctgac gcgccctgac gggcttgtct gctcccggca 3660

tccgcttaca gacaagctgt gaccgtctcc gggagctgca tgtgtcagag gttttcaccg 3720

tcatcaccga aacgcgcgag acgaaagggc ctcgtgatac gcctattttt ataggttaat 3780

gtcatgataa taatggtttc ttagacgtca ggtggcactt ttcggggaaa tgtgcgcgga 3840

acccctattt gtttattttt ctaaatacat tcaaatatgt atccgctcat gagacaataa 3900

ccctgataaa tgcttcaata atattgaaaa aggaagagta tgagtattca acatttccgt 3960

gtcgccctta ttcccttttt tgcggcattt tgccttcctg tttttgctca cccagaaacg 4020

ctggtgaaag taaaagatgc tgaagatcag ttgggtgcac gagtgggtta catcgaactg 4080

gatctcaaca gcggtaagat ccttgagagt tttcgccccg aagaacgttt tccaatgatg 4140

agcactttta aagttctgct atgtggcgcg gtattatccc gtattgacgc cgggcaagag 4200

caactcggtc gccgcataca ctattctcag aatgacttgg ttgagtactc accagtcaca 4260

gaaaagcatc ttacggatgg catgacagta agagaattat gcagtgctgc cataaccatg 4320

agtgataaca ctgcggccaa cttacttctg acaacgatcg gaggaccgaa ggagctaacc 4380

gcttttttgc acaacatggg ggatcatgta actcgccttg atcgttggga accggagctg 4440

aatgaagcca taccaaacga cgagcgtgac accacgatgc ctgtagcaat ggcaacaacg 4500

ttgcgcaaac tattaactgg cgaactactt actctagctt cccggcaaca attaatagac 4560

tggatggagg cggataaagt tgcaggacca cttctgcgct cggcccttcc ggctggctgg 4620

tttattgctg ataaatctgg agccggtgag cgtgggtctc gcggtatcat tgcagcactg 4680

gggccagatg gtaagccctc ccgtatcgta gttatctaca cgacggggag tcaggcaact 4740

atggatgaac gaaatagaca gatcgctgag ataggtgcct cactgattaa gcattggtaa 4800

ctgtcagacc aagtttactc atatatactt tagattgatt taaaacttca tttttaattt 4860

aaaaggatct aggtgaagat cctttttgat aatctcatga ccaaaatccc ttaacgtgag 4920

ttttcgttcc actgagcgtc agaccccgta gaaaagatca aaggatcttc ttgagatcct 4980

ttttttctgc gcgtaatctg ctgcttgcaa acaaaaaaac caccgctacc agcggtggtt 5040

tgtttgccgg atcaagagct accaactctt tttccgaagg taactggctt cagcagagcg 5100

cagataccaa atactgtcct tctagtgtag ccgtagttag gccaccactt caagaactct 5160

gtagcaccgc ctacatacct cgctctgcta atcctgttac cagtggctgc tgccagtggc 5220

gataagtcgt gtcttaccgg gttggactca agacgatagt taccggataa ggcgcagcgg 5280

tcgggctgaa cggggggttc gtgcacacag cccagcttgg agcgaacgac ctacaccgaa 5340

ctgagatacc tacagcgtga gctatgagaa agcgccacgc ttcccgaagg gagaaaggcg 5400

gacaggtatc cggtaagcgg cagggtcgga acaggagagc gcacgaggga gcttccaggg 5460

ggaaacgcct ggtatcttta tagtcctgtc gggtttcgcc acctctgact tgagcgtcga 5520

tttttgtgat gctcgtcagg ggggcggagc ctatggaaaa acgccagcaa cgcggccttt 5580

ttacggttcc tggccttttg ctggcctttt gctcacatgg ctcgacagat ccc 5633

## Description

### [Technical Field]

The present disclosure relates to an expression vector that includes a polynucleotide encoding a coagulation factor VIII mutant and has increased protein expression and a pharmaceutical composition containing the expression vector for preventing or treating hemorrhagic disease or hemorrhage.

### [Background Art]

Hemophilia A (HA) is one of severe genetic disorders. Hemophilia is a disorder related with a gene on X-chromosome and occurs in 1 out of 5000 males. It is caused by the variation of the plasma glycoprotein factor VIII (FVIII) which is an important component during blood coagulation. The factor VIII (hereinafter, FVIII) encodes 2351 amino acids and has six domains (A1-A2-B-A3-C1-C2). A heterodimer is formed by a heavy chain consisting of the A1, A2 and B domains and a light chain consisting of the A3, C1 and C2 domains.

Prior to the 1980s, patients with hemophilia received factor VIII extracted from the plasma of other people for treatment. However, this method had severe problems such as viral infection. Since the 1980s, full-length factor VIII produced in CHO cells, etc. through recombinant protein technology has been used in order to solve this problem. Later, as it is known that B domain-deleted factor VIII (F8-BDD) can be produced with high protein productivity with no difference in activity, recombinant proteins based on two-chain F8-BDD, which express a heavy chain and a light chain, respectively, have been developed and used. A recently developed truncated single-chain F8-BDD recombinant protein was proven to show 2- to 4-fold increased AUC as compared to the two-chain F8-BDD due to excellent in-vivo stability.

Although the factor VIII recombinant protein exhibits superior therapeutic effect, its concentration in the body should be consistently maintained at 5% or higher in order to prevent internal hemorrhage in patients with hemophilia. For this, the protein should be administered every day or 2-3 times a week. To overcome this disadvantage, a recombinant virus-based gene therapy agent was developed in the early 2000s. The recombinant virus-based gene therapy agent uses B-domain-deleted factor VIII (hereinafter, 'F8-BDD') with a size of smaller than 4.4 kb, rather than full-length factor VIII with a size of 7 kb or larger. In particular, most of therapeutic agents use the single-chain F8-BDD gene rather than the two-chain F8-BDD gene.

The inventors of the present disclosure have made efforts to develop a factor VIII gene therapy agent with further improved stability. In doing so, they have developed a single-chain F8-BDD mutant which exhibits improved stability and increased protein expression in eukaryotic cells as compared to the single-chain F8-BDD gene, and an expression vector expressing the same. In addition, they have identified that the expression and stability can be further improved by using an endogenous promoter such as elongation factor-1 alpha (EF-1α) and codon-optimizing (CO) the single-chain F8-BDD mutant, and have completed the present disclosure.

### [References of Related Art]

### [Patent Documents]

(Patent document 001) KR 10-1542752 B.

### [Disclosure]

### [Technical Problem]

The present disclosure is directed to providing a factor VIII mutant expression vector with increased protein expression.

The present disclosure is also directed to providing a pharmaceutical composition for preventing or treating hemorrhagic disease or hemorrhage, which contains the expression vector.

### [Technical Solution]

The present disclosure provides a factor VIII mutant expression vector including a single-chain polynucleotide encoding a factor VIII mutant wherein amino acids Asp784 to Arg1671 are deleted from factor VIII represented by SEQ ID NO 1.

In an exemplary embodiment of the present disclosure, the factor VIII mutant may have an amino acid sequence represented by SEQ ID NO 3.

In an exemplary embodiment of the present disclosure, the vector may be selected from a group consisting of pCDNA3.1, pGP and pEF.

In an exemplary embodiment of the present disclosure, the expression vector may have increased protein expression as compared to an expression vector including a polynucleotide encoding factor VIII represented by SEQ ID NO 1, B-domain-deleted factor VIII or single-chain factor VIII.

In addition, the present disclosure provides an expression system for expressing a factor VIII mutant, which includes the expression vector.

In addition, the present disclosure provides a pharmaceutical composition for preventing or treating hemorrhagic disease or hemorrhage, which contains the expression vector.

In an exemplary embodiment of the present disclosure, the hemorrhagic disease may be hemophilia A, hemophilia caused or complicated by an inhibitory antibody against factor VIII or factor Villa or hemophilia B.

In an exemplary embodiment of the present disclosure, the hemorrhagic disease may be one selected from a group consisting of neonatal coagulopathy, severe liver disease, thrombocytopenia, congenital deficiency of factor V, VII, X or XI, and von Willebrand disease with inhibitors against von Willebrand factor.

In an exemplary embodiment of the present disclosure, the hemorrhage may be caused by blood loss associated with a high-risk surgical procedure, traumatic blood loss, bone marrow transplantation or cerebral hemorrhage.

In an exemplary embodiment of the present disclosure, the pharmaceutical composition may be for gene therapy.

### [Advantageous Effects]

An expression vector including a polynucleotide encoding a factor VIII mutant of the present disclosure, with a part of the B-domain (residues 784-1667) and a part of the a3 region (residues 1668-1671) in factor VIII deleted, has remarkably increased protein expression.

### [Brief Description of Drawings]

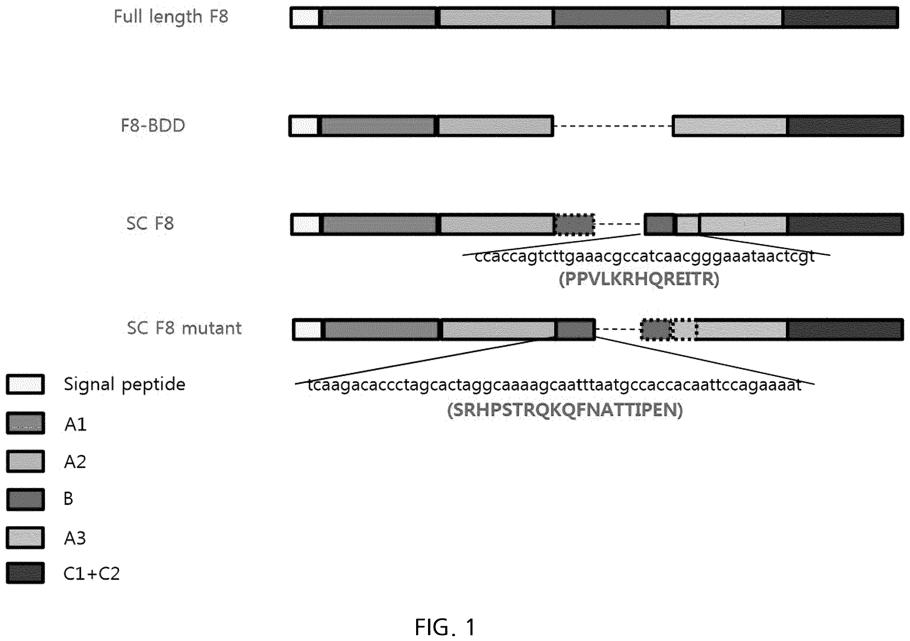

FIG. 1 schematically illustrates a process of designing a factor VIII mutant according to an exemplary embodiment of the present disclosure.

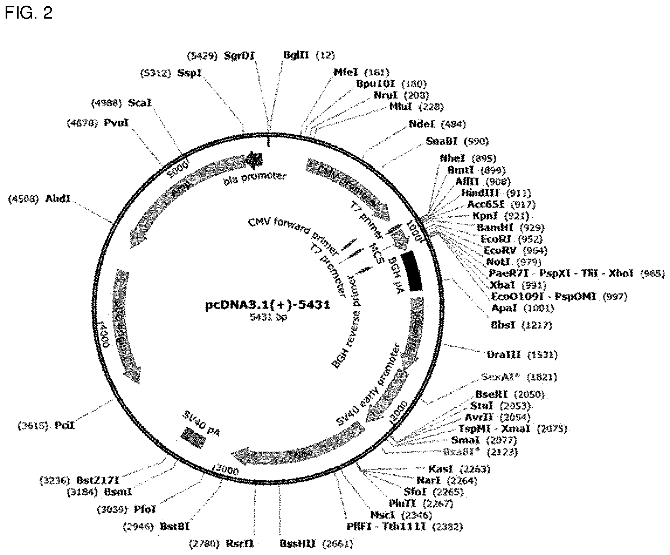

FIG. 2 shows the cleavage map of a pCDNA3.1 vector.

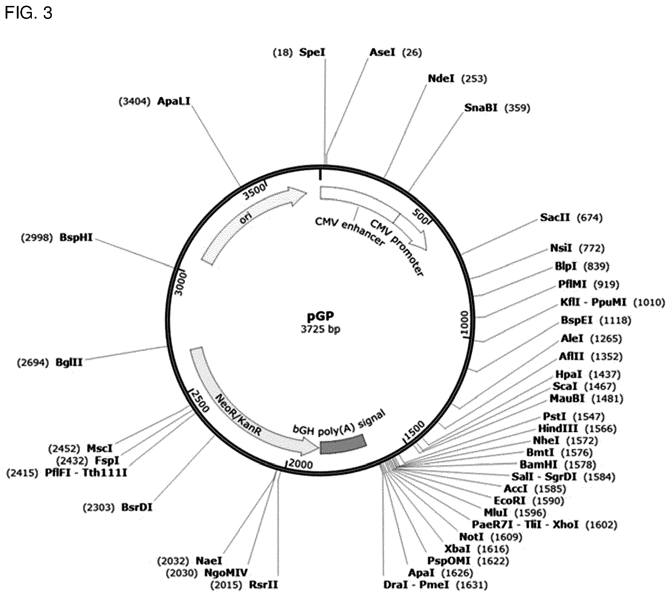

FIG. 3 shows the cleavage map of a pGP vector.

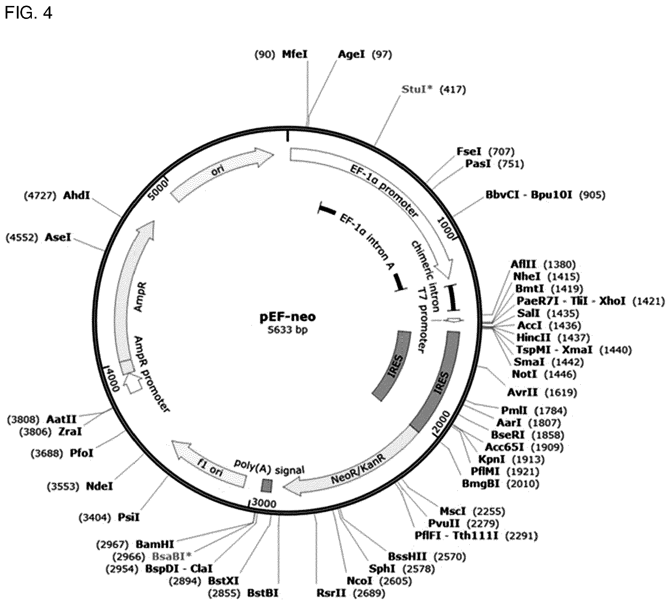

FIG. 4 shows the cleavage map of a pEF vector.
FIG. 5 shows a result of measuring protein expression level using a sample obtained from cells transfected with a pCDNA3.1 plasmid of Preparation Example 1.
FIG. 6 shows a result of measuring coagulant activity using a sample obtained from cells transfected with a pCDNA3.1 plasmid of Preparation Example 1.
FIG. 7 shows a result of measuring protein expression level using a sample obtained from cells transfected with a pGP plasmid of Preparation Example 2.
FIG. 8 shows a result of measuring protein expression level using a sample obtained from cells transfected with a pEP plasmid of Preparation Example 3.

### [Best Mode]

Hereinafter, the present disclosure is described in detail.

In an aspect, the present disclosure provides a factor VIII mutant expression vector including a single-chain polynucleotide encoding a factor VIII mutant wherein amino acids Asp784 to Arg1671 are deleted from factor VIII represented by SEQ ID NO 1.

### Factor VIII mutant

In the present specification, the terms "coagulation factor VIII", "factor VIII", "FVIII" and "F8" are used interchangeably. Mature human factor VIII consists of 2351 amino acids (including a signal peptide) and has the following domains:

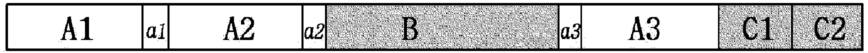

signal peptide: residues 1-19,
A1: residues 20-355,
A2: residues 392-729,
B: residues 760-1667,
A3: residues 1709-2038,
C1: residues 2039-2191 and
C2: residues 2192-2351.

In addition, there are three acidic domains a1 (356-391), a2 (730-759) and a3 (1668-1708). The acidic domain a3 is known to be involved in binding of the factor VIII molecule to von Willebrand factor (vWF) which plays an important role in blood coagulation. During secretion, the factor VIII is cleaved between the B-domain and the a3 acidic domain, resulting in a heterodimer polypeptide. The factor VIII heterodimer is composed of a light chain (including A3, C1 and C2) and a heavy chain (including A1, A2 and B) of variable size. The heavy chain is heterologous due to limited proteolysis within the B-domain. In case of a heterodimer B-domain-deleted factor VIII, the "heavy chain" includes A1 and A2, but lacks a part or all of the B-domain.

The amino acid sequence of mature wild-type human coagulation factor VIII is shown in SEQ ID NO 1. The reference number to an amino acid position in a particular sequence refers to the location of the corresponding amino acid in the wild-type FVIII protein and does not exclude the presence of mutations (deletion, insertion and/or substitution) at other locations in the sequence. A DNA sequence encoding the SEQ ID NO 1 is SEQ ID NO 2.

The "coagulation factor VIII" includes not only the wild-type coagulation factor VIII but also a derivative of the wild-type coagulation factor VIII having the procoagulant activity of the wild-type coagulation factor VIII. The derivative may have deletion, insertion and/or addition as compared to the amino acid sequence of the wild-type factor VIII. A preferred derivative is an FVIII molecule from which all or a part of the B-domain has been deleted. The amino acid positions indicated throughout the present specification always refer to the positions of the respective amino acids in the mature full-length wild-type factor VIII (including a signal peptide cleavage).

In the present specification, the term "mutant" includes conservative or non-conservative substitution, insertion or deletion of an amino acid sequence, a nucleic acid sequence, etc. This variation does not substantially alter the active site or active domain which confers the biological activities of FVIII.

The factor VIII mutant according to the present disclosure refers to one wherein amino acids Asp784 to Arg1671 are deleted from factor VIII represented by SEQ ID NO 1. The mutant is a single-chain factor VIII mutant. The factor VIII mutant is one wherein a part of the B-domain (residues 784-1667) and a part of the a3 region (residues 1668-1671) are deleted, and the mutant has increased protein expression and improved coagulant activity and stability as compared to factor VIII, B-domain-deleted factor VIII and single-chain factor VIII. The factor VIII mutant has an amino acid sequence represented by SEQ ID NO 3.

The "single-chain factor VIII" refers to a factor VIII molecule which exists as a single polypeptide chain without being cleaved into two chains (e.g., a heavy chain and a light chain) during secretion from cells expressing the factor VIII molecule.

### Polynucleotide

The present disclosure also relates to a polynucleotide encoding the factor VIII mutant having an amino acid sequence represented by SEQ ID NO 3.

In the present specification, the term "polynucleotide" refers to any polyribonucleotide or polydeoxyribonucleotide which may be an unmodified RNA or DNA, or a modified RNA or DNA. The polynucleotide of the present disclosure may be a single-chain DNA or RNA. The term "polynucleotide" used in the present specification includes a DNA or RNA containing a modified base and/or an unusual base, e.g., inosine. It is obvious that various modifications that provide known useful purposes can be made to the DNA or RNA. The term "polynucleotide" used in the present specification also includes a chemically, enzymatically or metabolically modified polynucleotide.

Those skilled in the art will understand that the factor VIII mutant may be encoded by several polynucleotides due to the degeneracy of the genetic code. That is to say, it is understood that a polynucleotide sequence encoding the factor VIII mutant that can be used in the present disclosure also includes a nucleotide sequence that exhibits substantial identity to the amino acid sequence represented by SEQ ID NO 3.

Specifically, the polynucleotide of the present disclosure may be an isolated polynucleotide. The "isolated" polynucleotide means a polynucleotide that is substantially free of other nucleic acid sequences, such as chromosomal and extrachromosomal DNA and RNA, although not being limited thereto. The isolated polynucleotide may be purified from a host cell. Common nucleic acid purification methods known to those skilled in the art may be used obtain the isolated polynucleotide. The term also includes a recombinant polynucleotide and a chemically synthesized polynucleotide.

### Expression system for expressing a factor VIII mutant

### Expression vector

In another aspect, the present disclosure provides an expression vector including a single-chain polynucleotide encoding the factor VIII mutant (hereinafter, referred to as a 'factor VIII mutant expression vector').

In the present specification, the term "expression" refers to production of the factor VIII mutant in a cell.

In the present specification, the term "expression vector" refers to a vector capable of expressing the factor VIII mutant in a suitable host cell, and refers to a gene construct containing an essential regulatory element to which a gene insert is operably linked in such a manner as to be expressed.

In the present specification, the term "operably linked" refers to functional linkage between a nucleic acid expression control sequence and a polynucleotide encoding the factor VIII mutant to perform a general function. For example, a promoter may be operably linked to a polynucleotide encoding the factor VIII mutant to affect the expression of the polynucleotide. The operable linkage with a recombinant vector may be achieved using a gene recombination technique well known in the art. For site-specific DNA cleavage and linkage, enzymes generally known in the art may be used.

The expression vector of the present disclosure is constructed using a plasmid, a vector or a viral vector, although not being limited thereto. A suitable expression vector may include a regulatory element such as a promoter, an operator, a start codon, a stop codon, a polyadenylation signal and an enhancer, and may be prepared in various manners depending on the intended use. The promoter of the vector may be constitutive or inducible.

Specifically, the expression vector may be prepared by using a vector selected from a group consisting of pCDNA3.1, pGP and pEF in terms of effect. Specifically, the factor VIII mutant expression vector has remarkably increased protein expression as compared to a polynucleotide encoding factor VIII represented by SEQ ID NO 1, B-domain-deleted factor VIII or single-chain factor VIII. In particular, it was confirmed that protein expression and stability can be further improved by using an endogenous promoter such as an EF1a promoter, etc. and by codon-optimizing (CO) the single-chain F8-BDD mutant.

In order to produce the factor VIII mutant at a high level in a host cell, an appropriate regulatory element is necessary in a recombinant expression vector that can be proliferated in various expression systems according to methods known to those skilled in the art, together with an assembly of modified cDNA. Effective transcriptional control elements may be derived from viruses having animal cells as natural hosts or from chromosomal DNA of animal cells. Specifically, a promoter-enhancer combination derived from the long terminal repeat of simian virus 40, adenovirus, BK polyomavirus, human cytomegalovirus or Rous sarcoma virus, or a promoter-enhancer combination including a strongly constitutively transcribed gene in animal cells, such as beta-actin or GRP78, may be used. In order to achieve stable, high levels of mRNA transcribed from cDNA, the transcriptional unit should contain in its 3'-proximal part a DNA region encoding a transcriptional termination-polyadenylation sequence. Specifically, this sequence is derived from the simian virus 40 early transcriptional region, rabbit beta-globin gene or human tissue plasminogen activator gene.

### Expression of factor VIII mutant

The expression vector may be transfected into a suitable host cell and, as a result, may lead to the expression of the factor VIII mutant of the present disclosure and the production of a functional protein.

In another aspect, the present disclosure provides a host cell including the polynucleotide or the expression vector.

The host cell of the present disclosure may be used in a method for producing the factor VIII mutant of the present disclosure. The method may include: (a) a step of culturing the host cell under a condition where the factor VIII mutant can be expressed; and (b) a step of recovering the factor VIII mutant from the host cell or a culture medium.

Specifically, for expression of the factor VIII mutant, a nucleotide such as cDNA is incorporated into the genome of a suitable host cell. Specifically, the host cell should be an animal cell of vertebrate origin in order to ensure correct folding, disulfide bond formation, asparagine-linked glycosylation and other post-translational modifications and to ensure secretion into a culture medium. Examples of other post-translational modifications include O-sulfation of tyrosine and proteolytic processing of a nascent polypeptide chain. Examples of the cell that can be used include monkey COS cells, mouse L cells, mouse C127 cells, hamster BHK-21 cells, human embryonic kidney 293 cells, and hamster CHO cells.

A recombinant expression vector encoding the corresponding cDNA may be introduced into an animal cell in different ways. For example, the recombinant expression vector can be constructed from vectors based on viruses. Examples include the vectors based on baculovirus, vaccinia virus and adenovirus, specifically bovine papillomavirus.

The transcriptional unit encoding the corresponding DNA can also be introduced into an animal cell together with another recombinant gene which may function as a dominant selection marker in the cell in order to facilitate the isolation of specific cell clones integrated to the recombinant DNA in the genome. Examples of the dominant selection marker gene include Tn5 aminoglycoside phosphotransferase conferring resistance to geneticin (G418), hygromycin phosphotransferase conferring resistance to hygromycin, and puromycin acetyltransferase conferring resistance to puromycin. The recombinant expression vector encoding such a selection marker may be present on the same vector as the vector encoding the cDNA of the desired protein, or may be encoded on a separate vector which is simultaneously introduced and integrated into the genome of the host cell, frequently resulting in a strong physical linkage between different transcriptional units.

Other types of the selection marker gene that can be used tougher with the cDNA of the desired protein are based on various transcriptional units encoding dihydrofolate reductase (DHFR). After introduction of this type of gene into cells lacking endogenous DHFR activity, typically CHO cells (DUKX-B11, DG-44), it will enable the cells to grow in a medium lacking nucleosides. For example, the medium may be Ham's F12 without hypoxanthine, thymidine and glycine. The DHFR gene can be introduced together with the factor VIII cDNA transcriptional unit into CHO cells, being linked on the same vector or on different vectors, thus creating DHFR-positive cells producing a recombinant protein.

If the cells are proliferated in the presence of the cytotoxic DHFR inhibitor methotrexate, new cells resistant to methotrexate will be produced. These cells can produce a recombinant protein at an increased rate due to the amplified number of transcriptional units linked to DHFR. When these cells are proliferated while increasing the concentration of methotrexate (from 1 to 10000 nM), new cells producing the desired protein can be obtained with very high rate.

The cells producing the desired protein can be proliferated on a large scale, through suspension culture or on various solid supports. Examples of the support include a microcarrier based on dextran or collagen matrix, or a solid support in the form of a hollow fiber or various ceramic materials. When proliferated through suspension culture or on a microcarrier, the culturing of the cells can be performed by bath culture or perfusion culture whereby conditioned media are produced continuously over extended periods of time. Thus, according to the present disclosure, the above-described cells are well suited for the development of an industrial process for the production of the desired recombinant mutant protein.

### Pharmaceutical composition

In addition, the present disclosure provides a pharmaceutical composition for preventing or treating hemorrhagic disease or hemorrhage, which contains the expression vector described above.

The hemorrhagic disease may be hemophilia A, hemophilia caused or complicated by an inhibitory antibody against factor VIII or factor Villa, or hemophilia B. In addition, the hemorrhagic disease may be one selected from a group consisting of neonatal coagulopathy, severe liver disease, thrombocytopenia, congenital deficiency of factor V, VII, X or XI, and von Willebrand disease with inhibitors against von Willebrand factor.

And, the hemorrhage may be caused by blood loss associated with a high-risk surgical procedure, traumatic blood loss, bone marrow transplantation or cerebral hemorrhage.

The pharmaceutical composition of the present disclosure may be for gene therapy.

### Purification of factor VIII mutant

The factor VIII mutant which is secreted from the above-described cells and accumulated in the culture thereof can be concentrated and purified by a variety of biochemical and chromatographic methods, including methods utilizing differences in size, charge, hydrophobicity, solubility, specific affinity, etc. between the desired protein and other substances in the cell culture medium.

Specifically, the factor VIII mutant of the present disclosure may be purified to a purity of 80% or higher, more specifically 95% or higher. In particular, it is desired that the mutant is in a pharmaceutically pure state, with a purity higher than 99.9% with respect to contaminating macromolecules, particularly other proteins and nucleic acids, and free of infectious and pyrogenic agents. Specifically, the isolated or purified mutant of the present disclosure is substantially free of other unrelated polypeptides.

As an example of such purification, after adsorbing the factor VIII mutant onto a solid support, followed by washing and desorption, the protein may be further purified by various chromatographic techniques based on its properties. The sequence of purification steps is selected according to the ability or selectivity of the steps, the stability of the support, or other aspects. Preferred purification steps include, for example, ion-exchange chromatography, immunoaffinity chromatography, affinity chromatography, hydrophobic interaction chromatography, dye chromatography and size exclusion chromatography steps, although not being limited thereto.

To minimize the theoretical risk of viral contamination, additional steps may be included in the method to effectively inactivate or eliminate viruses. Such steps are, for example, heat treatment in liquid or solid state, solvent and/or surfactant treatment, light irradiation in the visible or UV spectrum, gamma irradiation or nanofiltration.

The modified polynucleotide (e.g., DNA) the present disclosure may also be incorporated into a transfer vector used in human gene therapy.

The various aspects described in the present specification may be combined with one another. Hereinafter, the present disclosure will be described in more detail by way of examples. The description of certain aspects of the present disclosure will be set forth in connection with the accompanying drawings.

### Formulation

The inserted protein described in the present disclosure can be formulated into a pharmaceutical preparation for therapeutic use. The purified protein is dissolved in a common physiologically acceptable aqueous buffer solution and a pharmaceutical excipient may be optionally added thereto to provide a pharmaceutical preparation.

Pharmaceutical carriers and excipients as well as suitable pharmaceutical formulations are well known in the art (e.g., "Pharmaceutical Formulation of Peptides and Proteins", Frokjaer et al., Taylor & Francis (2000) or "Handbook of Pharmaceutical Excipients", 3rd edition, Kibbe et al., Pharmaceutical Press (2000)). In particular, a pharmaceutical composition containing the mutant of the present disclosure can be formulated into a lyophilized form or a stable liquid form. The mutant of the present disclosure can be lyophilized through a variety of procedures known in the art. The lyophilized formulation may be reconstituted prior to use by addition of one or more pharmaceutically acceptable diluent, such as injectable sterile water or sterile saline.

The formulation of the composition is delivered to a subject by any pharmaceutically appropriate means of administration. A variety of delivery systems are known and can be used to administer the composition by any convenient route. Typically, the composition of the present disclosure is administered systemically. In the case of systemic administration, the inserted protein of the present disclosure is formulated for parenteral (e.g., intravenous, subcutaneous, intramuscular, intraperitoneal, intracerebral, intrapulmonary, intranasal or transdermal) delivery or intestinal (e.g., oral, vaginal or rectal) delivery. The most preferred route of administration is intravenous and subcutaneous administration. These formulations may be administered continuously by infusion or bolus injection. Some formulations include sustained-release systems.

The inserted protein of the present disclosure may be administered to a patient in a therapeutically effective amount that does not reach a dose that causes unacceptable side effects and that is sufficient to produce the desired effect while preventing or reducing the severity or development of the condition or symptom to be treated. The exact dosage depends on a variety of factors, including signs, formulations and administration method, and each indication should be determined through preclinical and clinical trials.

The pharmaceutical composition of the present disclosure may be administered alone or in combination with other therapeutic agents. Such agents may be included in the same formulation. An example of such a formulation is von Willebrand factor.

### Therapeutic method

The present disclosure also relates to a method for treating a subject suffering from hemophilia A, hemophilia B or a hemorrhagic disease such as acquired hemophilia. The therapeutic method may include a step of administering an effective amount of a pharmaceutical composition containing the expression vector of the present disclosure to a subject in need thereof. Alternatively, it may include a step of administering an effective amount of the host cell of the present disclosure to the subject.

According to an exemplary embodiment of the present disclosure, the factor VIII mutant of the present disclosure may be administered in an amount of 10 ng to100 mg, and a polynucleotide encoding the protein may be administered in an amount of 1 µg to 100 mg. When the factor VIII mutant or the polynucleotide encoding the same is administered more than once, each administration dosage may be the same or different.

Hereinafter, the present disclosure will be described in detail through examples. However, the following examples are for illustrative purposes only and the scope of the present disclosure is not limited by the examples.

### Examples

### Example 1. Construction of factor VIII mutant (F8M)

A fragment 1 represented by SEQ ID NO 5 was prepared through polymerase chain reaction (PCR) by using a full-length factor VIII gene represented by SEQ ID NO 1 as a template and using primers 1 and 2. The PCR was conducted by preparing a mixture solution of 2 µL of the template DNA, 1 µL of each primer at 10 pmol/µL, 2.5 µL of 2.5 mM dNTP, 1 µL of a Pfu enzyme mix (Enzynomics, Korea) and 2.5 µL of a 10x buffer by adding sterilized triply distilled water to 50 µL and repeating 40 cycles of 30 seconds at 95 ºC, 30 seconds at 60 ºC and 30 seconds at 72 ºC. A fragment 2 represented by SEQ ID NO 6 was prepared in the same manner through PCR by using primers 8 and 3. Then, a single-chain factor VIII mutant was prepared by conducing overlapping PCR with the fragments 1 and 2 using primers 9 and 10. The overlapping PCR was conducted by in the same manner as described above except for adding 2 µL of the DNA fragment.

### Example 2. Construction of factor VIII mutant CO (F8M CO)

After establishing a codon-optimized base sequence on the basis of the base sequence of the factor VIII mutant prepared in Example 1 using the OptimumGene™ algorithm, a factor VIII mutant CO represented by SEQ ID NO 4 was synthesized by Genscript on the basis of the base sequence.

### Comparative Example 1. Construction of factor VIII (F8)

A full-length factor VIII (F8) gene represented by SEQ ID NO 1 (NM_000132.3) was purchased from Origene Technologies (MD, USA).

### Comparative Example 2. Construction of B-domain-deleted factor VIII (F8 BDD)

A fragment 3 represented by SEQ ID NO 7 was prepared through polymerase chain reaction (PCR) by using a full-length factor VIII gene represented by SEQ ID NO 1 as a template and using primers 1 and 4. The PCR was conducted by preparing a mixture solution of 2 µL of the template DNA, 1 µL of each primer at 10 pmol/µL, 2.5 µL of 2.5 mM dNTP, 1 µL of a Pfu enzyme mix (Enzynomics, Korea) and 2.5 µL of a 10x buffer by adding sterilized triply distilled water to 50 µL and repeating 40 cycles of 30 seconds at 95 ºC, 30 seconds at 60 ºC and 30 seconds at 72 ºC. A fragment 4 represented by SEQ ID NO 8 was prepared in the same manner through PCR by using primers 8 and 5. Then, a B-domain-deleted factor VIII (F8 BDD) was prepared by conducing overlapping PCR with the fragments 3 and 4 using primers 9 and 10. The overlapping PCR was conducted by in the same manner as described above except for adding 2 µL of the DNA fragment.

### Comparative Example 3. Construction of single-chain factor VIII (sc F8)

A fragment 5 represented by SEQ ID NO 9 was prepared through polymerase chain reaction (PCR) by using a full-length factor VIII gene represented by SEQ ID NO 1 as a template and using primers 1 and 6. The PCR was conducted by preparing a mixture solution of 2 µL of the template DNA, 1 µL of each primer at 10 pmol/µL, 2.5 µL of 2.5 mM dNTP, 1 µL of a Pfu enzyme mix (Enzynomics, Korea) and 2.5 µL of a 10x buffer by adding sterilized triply distilled water to 50 µL and repeating 40 cycles of 30 seconds at 95 ºC, 30 seconds at 60 ºC and 30 seconds at 72 ºC. A fragment 6 represented by SEQ ID NO 10 was prepared in the same manner through PCR by using primers 8 and 7. Then, a single-chain factor VIII mutant (sc F8) was prepared by conducing overlapping PCR with the fragments 5 and 6 using primers 9 and 10. The overlapping PCR was conducted by in the same manner as described above except for adding 2 µL of the DNA fragment.

The primers used in Examples and Comparative Examples are described in Table 1.

**[Table 1]**

| Primer No. | Primer name | Base sequence |
|---|---|---|
| 1 | F8 (F) | ATGCAAATAGAGCTCTCCACCTGCTTCTTT |
| 2 | F8M-1 (R) | TCTGACTGAAGAGTAGTATTTTCTGGAATTGTGGTG GCATTAAAT |
| 3 | F8M-2 (F) | CCACAATTCCAGAAAATACTACTCTTCAGTCAGATC AAGAGGAAATTGAC |
| 4 | F8 BDD-1 (R) | AGAGTAGTACGAGTTATTTCTCTTGGTTCAATGGCA T |
| 5 | F8 BDD-2 (F) | GAACCAAGAGAAATAACTCGTACTACTCTTCAGTCA GA |
| 6 | F8 Sc-1 (R) | TCAAGACTGGTGGATTCTGGGAGAAGCTTCTTGGTT C |
| 7 | F8 Sc-2 (F) | CTTCTCCCAGAATCCACCAGTCTTGAAACGCCA |
| 8 | F8 (R) | TCAGTAGAGGTCCTGTGCCTCGCAGCCCAG |
| 9 | F8 final (F) | GCTAGCATGCAAATAGAGCTCTCCACCTGC |
| 10 | F8 final (R) | GCGGCCGCTCAGTAGAGGTCCTGTGCCTCGCA |
| 11 | pGP (F) | GACGAATTCACGCGTCTCGAGGCGGCCGCTCTAGA |
| | | GGGCCCGTTTAAA |
| 12 | pGP (R) | GACGAATTCGTCGACGGATCCGCTAGCAAGCTTCG TGTCAAGGACGGT |

### Preparation Examples

### Preparation Example 1. Construction of expression vector (pCDNA3.1)

Each of pCDNA3.1 (represented by SEQ ID NO 12) purchased from Genscript and the proteins prepared in Examples and Comparative Examples was cleaved with Nhel and Notl enzymes for 1 hour and then fragments were separated by electrophoresing on agarose gel. The separated fragments were ligated for 30 minutes using T4 ligase, and then introduced into *E. coli.* After culturing the *E. coli* overnight, DNA was isolated from the cultured colony on the next day through mini-preparation and then investigated using Nhel and Notl.

### Preparation Example 2. Construction of expression vector (pGP)

After synthesizing a pCK vector by referring to Lee, et al. (Lee Y, et al. Improved expression of vascular endothelial growth factor by naked DNA in mouse skeletal muscles: implication for gene therapy of ischemic diseases. Biochem. Biophys. Res. Commun. 2002; 272(1): 230-235), fragments were obtained by conducting PCR as described above using primers 11 and 12 described in Table 1. Using an EcoRI enzyme, the fragments were reacted at 37ºC for 1 hour, and then DNA was purified using an Expin Gel SV kit (GeneAll, Korea). Then, after ligating for 30 minutes using T4 ligase and introducing into *E. coli,* and then culturing the *E*. *coli* overnight, a pGP vector represented by SEQ ID NO 13 was constructed by isolating DNA from the cultured colony on the next day through mini-preparation.

Each of the prepared pGP vector and the fragments prepared in Examples and Comparative Examples was cleaved with Nhel and Notl enzymes for 1 hour and then fragments were separated by electrophoresing on agarose gel. The separated fragments were ligated for 30 minutes using T4 ligase, and then introduced into *E*. *coli.* After culturing the *E. coli* overnight, DNA was isolated from the cultured colony on the next day through mini-preparation and then investigated using Nhel and Notl.

### Preparation Example 3. Construction of expression vector (pEF)

Each of a pEF vector (represented by SEQ ID NO 14) purchased from ThermoFisher Scientifics and the DNA fragments prepared in Examples and Comparative Examples was cleaved with Nhel and Notl enzymes for 1 hour and then fragments were separated by electrophoresing on agarose gel. The separated fragments were ligated for 30 minutes using T4 ligase, and then introduced into *E*. *coli.* After culturing the *E. coli* overnight, DNA was isolated from the cultured colony on the next day through mini-preparation and then investigated using Nhel and Notl.

The prepared expression vectors are summarized in Table 2.

**[Table 2]**

| Expression vector | Expressed protein | Symbol | Vector |
|---|---|---|---|
| Control 1 | - | - | pCDNA3.1 (Preparation Example 1) |
| Example 1-1 | Factor VIII mutant | F8M | |
| Example 2-1 | Codon-optimized factor VIII mutant | F8M CO | |
| Comparative | Factor VIII | F8 | |
| Example 1-1 | | | |
| Comparative Example 2-1 | B-domain-deleted factor VIII | F8-BDD | |
| Comparative Example 3-1 | Single-chain factor VIII | scF8 | |
| Control 2 | - | - | pGP (Preparation Example 2) |
| Example 1-2 | Factor VIII mutant | F8M | |
| Example 2-2 | Codon-optimized factor VIII mutant | F8M CO | |
| Comparative Example 1-2 | Factor VIII | F8 | |
| Comparative Example 2-2 | B-domain-deleted factor VIII | F8-BDD | |
| Comparative Example 3-2 | Single-chain factor VIII | scF8 | |
| Control 3 | - | - | pEF (Preparation Example 3) |
| Example 1-3 | Factor VIII mutant | F8M | |
| Example 2-3 | Codon-optimized factor VIII mutant | F8M CO | |
| Comparative Example 1-3 | Factor VIII | F8 | |
| Comparative Example 2-3 | B-domain-deleted factor VIII | F8-BDD | |
| Comparative Example 3-3 | Single-chain factor VIII | scF8 | |

### Test Examples

### Preparation of sample

The expression vectors prepared in Preparation Examples were purified according to a standard protocol (Qiagen). First, after transforming each plasmid DNA into *E. coli,* the *E*. *coli* was cultured overnight at 37 ºC. Centrifugation was conducted after lysing the *E. coli* cells with P1, P2 and P3. The obtained supernatant was passed through a maxi-prep column by gravity flow and a supernatant containing the plasmid DNA was obtained by using an elution buffer. After adding 0.7 volume equivalent of isopropanol, expression vectors were obtained by centrifuging at 12,000 rpm for 30 minutes using a high-speed centrifuge. Each expression vector was transfected into 5x10⁵ 293T cells (ATCC CRL1573) using JetPEI (Polyplus, USA) according to the manufacturer's instructions. At 6 hours, the medium was replaced with a serum-free medium. Then, after culturing the cells at 37 ºC and recovering supernatants on days 2 and 3, centrifugation was performed at 12,000 rpm for 5 minutes and the supernatant was stored at -80 ºC.

### Test Example 1. Protein expression level and activity of cells including expression vector (pCDNA3.1)

Protein expression level and biological activity were measured for samples obtained from the cells transfected with the expression vectors of Preparation Example 1.

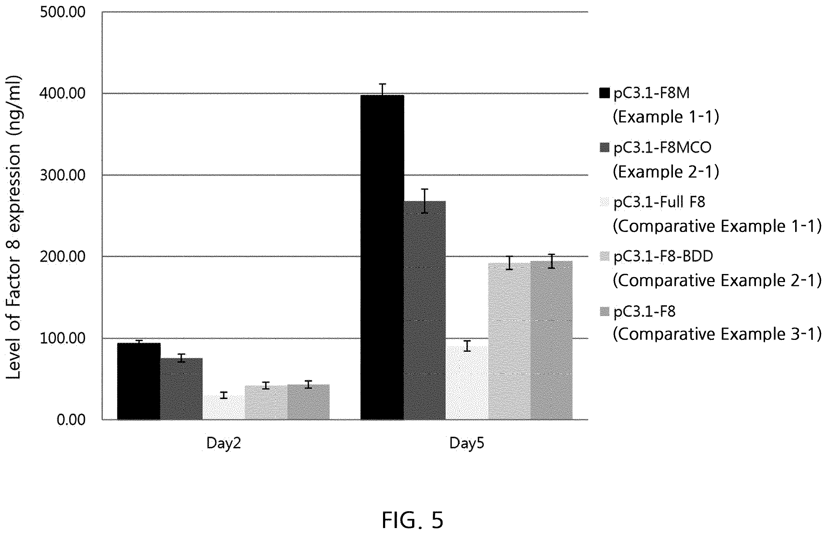

The protein expression level was measured using an ELISA kit (Stago Asserchrom VIII:Ag, France). First, the sample on day 2 was diluted to 1/5 and the sample on day 5 was diluted to 1/50. Then, 200 µL of a reference standard and the sample were added to a 96-well plate and incubated at 18-25 ºC for 2 hours. After washing 5 times, the resultant was incubated with a secondary antibody at 18-25 ºC for 2 hours. After washing 5 times and reacting with a TMB solution for 5 minutes, the reaction was stopped with a 1 M sulfuric acid solution and absorbance was measured at 450 nm using an ELISA reader. The result is shown in FIG. 5 and Table 3.

**[Table 3]**

| | Protein expression level (ng/mL) | |
|---|---|---|
| | Day 2 | Day 5 |
| Control 1 | - | - |
| Example 1-1 | 93.52 | 397.28 |
| Example 2-1 | 75.72 | 268.15 |
| Comparative Example 1-1 | 30.20 | 90.52 |
| Comparative Example 2-1 | 42.08 | 192.40 |
| Comparative Example 3-1 | 43.45 | 194.51 |

As seen from FIGS. 5 and Table 3, the pCDNA3.1-F8 mutant (Example 1-1) showed significantly high protein expression level on both days 2 and 5. In particular, the difference in expression level was more distinct on day 5 than on day 2. In contrast, protein expression was not observed for the pCDNA3.1 vehicle (control 1).

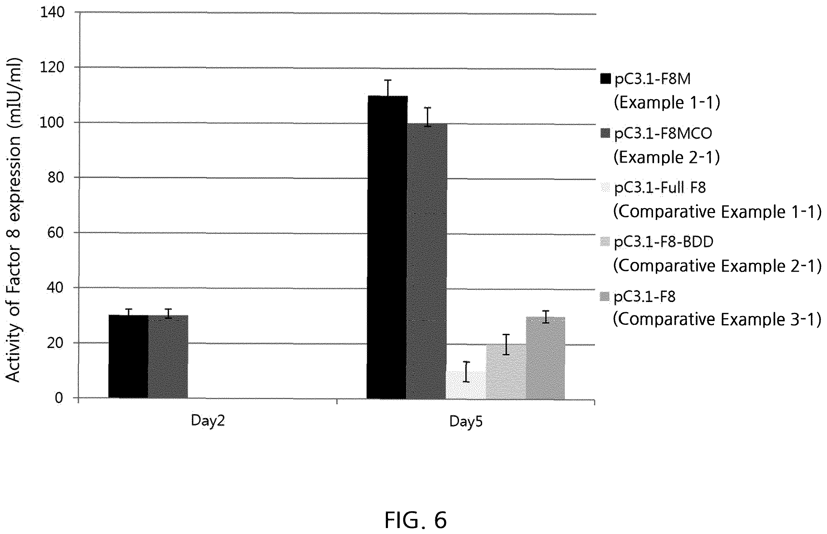

In addition, biological activity was measured using a coagulant activity assay kit (Stago, France). First, 1 IU/mL of a reference standard was serially diluted with an Owren-Koller buffer to obtain solutions with concentrations of 0.51, 0.25 and 0.13 IU/mL. Then, after adding 0.025 M CaCl₂, an APTT reagent activator and factor VIII-deficient plasma to the sample or the standard solution and incubating at 37 ºC, measurement was made using a coagulation analyzer (Stago Compact, France). The measurement result was normalized to the activity of the reference standard and the result is shown in FIG. 6 and Table 4.

**[Table 4]**

| | Coagulant activity (mIU/ml) | |
|---|---|---|
| | Day 2 | Day 5 |
| Control 1 | 0 | 0 |
| Example 1-1 | 30 | 110 |
| Example 2-1 | 30 | 100 |
| Comparative Example 1-1 | 0 | 0 |
| Comparative Example 2-1 | 0 | 20 |
| Comparative Example 3-1 | 0 | 30 |

As seen from FIG. 6 and Table 4, the CDNA3.1-F8 mutant (Example 1-1) showed remarkably high coagulant activity.

### Test Example 2. Protein expression level of cells including expression vector (pGP)

Protein expression level was measured for samples obtained from the cells transfected with the expression vectors of Preparation Example 2.

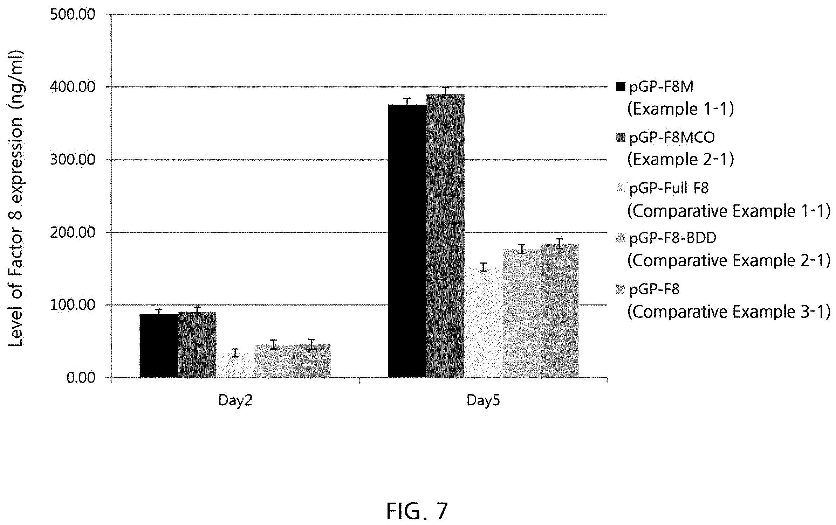

The protein expression level was measured using an ELISA kit (Stago Asserchrom VIII:Ag, France). First, the sample on day 2 was diluted to 1/5 and the sample on day 5 was diluted to 1/50. Then, 200 µL of a reference standard and the sample were added to a 96-well plate and incubated at 18-25 ºC for 2 hours. After washing 5 times, the resultant was incubated with a secondary antibody at 18-25 ºC for 2 hours. After washing 5 times and reacting with a TMB solution for 5 minutes, the reaction was stopped with a 1 M sulfuric acid solution and absorbance was measured at 450 nm using an ELISA reader. The result is shown in FIG. 7 and Table 5.

**[Table 5]**

| | Protein expression level (ng/mL) | |
|---|---|---|
| | Day 2 | Day 5 |
| Control 2 | - | - |
| Example 1-2 | 44.76 | 179.05 |
| Example 2-2 | 67.44 | 269.67 |
| Comparative Example 1-2 | 21.13 | 76.25 |
| Comparative Example 2-2 | 24.60 | 94.80 |
| Comparative Example 3-2 | 31.00 | 120.90 |

As seen from FIG. 7 and Table 5, the pGP-F8 mutant (Example 1-2) showed significantly high protein expression level on both days 2 and 5. In particular, the difference in expression level was more distinct on day 5 than on day 2. In contrast, protein expression was not observed for the pGP vehicle (control 2).

### Test Example 3 Protein expression level of cells including expression vector (pEF)

Protein expression level was measured for samples obtained from the cells transfected with the expression vectors of Preparation Example 3.

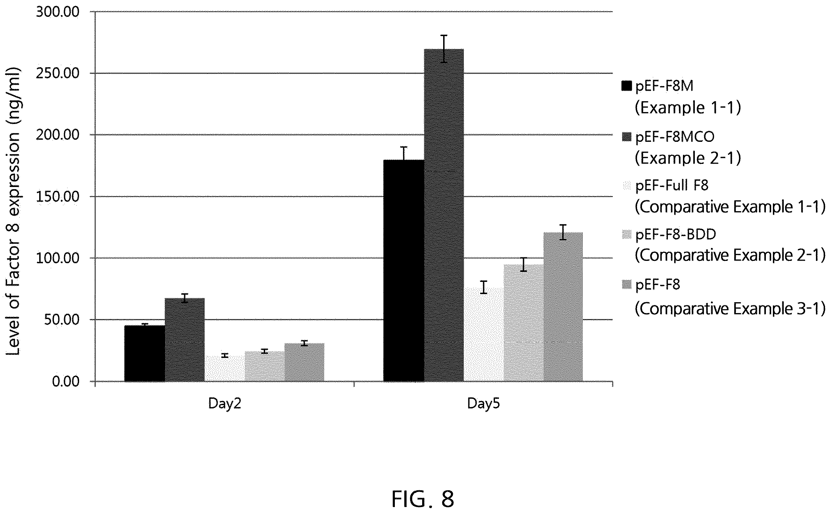

The protein expression level was measured using an ELISA kit (Stago Asserchrom VIII:Ag, France). First, the sample on day 2 was diluted to 1/5 and the sample on day 5 was diluted to 1/50. Then, 200 µL of a reference standard and the sample were added to a 96-well plate and incubated at 18-25 ºC for 2 hours. After washing 5 times, the resultant was incubated with a secondary antibody at 18-25 ºC for 2 hours. After washing 5 times and reacting with a TMB solution for 5 minutes, the reaction was stopped with a 1 M sulfuric acid solution and absorbance was measured at 450 nm using an ELISA reader. The result is shown in FIG. 8 and Table 6.

**[Table 6]**

| | Protein expression level (ng/mL) | |
|---|---|---|
| | Day 2 | Day 5 |
| Control 3 | - | - |
| Example 1-3 | 87.28 | 375.30 |
| Example 2-3 | 90.22 | 389.74 |
| Comparative Example 1-3 | 34.24 | 152.08 |
| Comparative Example 2-3 | 45.70 | 176.96 |
| Comparative Example 3-3 | 46.04 | 184.36 |

As seen from FIG. 8 and Table 6, the pEF-F8 mutant (Example 1-3) showed significantly high protein expression level on both days 2 and 5. In particular, the difference in expression level was more distinct on day 5 than on day 2. In contrast, protein expression was not observed for the pEF vehicle (control 3).

From the above results, it was confirmed that the factor VIII mutant (F8M) may exhibit higher protein expression as compared to factor VIII (F8). In particular, it is thought that the factor VIII mutant (F8M) protein has improved stability as compared to the factor VIII (F8) because the protein expression level is higher on day 5 than on day 2. In addition, the coagulant activity on day 5 was higher (about 2.5 times) for the factor VIII mutant (F8M) as compared to factor VIII (F8). Through this, it can be seen that the factor VIII mutant (F8M) gene of the present disclosure therapy agent expresses proteins with high coagulant activity at higher levels. Additionally, it was observed that the factor VIII mutant (F8M) exhibits further improved protein expression level and stability on day 5 when codon-optimized with a pEF vector having an EF1a promoter (FIG. 8).

While the exemplary embodiments have been shown and described, it will be understood by those skilled in the art that various changes in form and details may be made thereto without departing from the spirit and scope of this disclosure as defined by the appended claims.

## Claims

1. A factor VIII mutant expression vector comprising a single-chain polynucleotide encoding a factor VIII mutant wherein amino acids Asp784 to Arg1671 are deleted from factor VIII represented by SEQ ID NO 1.

2. The factor VIII mutant expression vector according to claim 1, wherein the factor VIII mutant has an amino acid sequence represented by SEQ ID NO 3.

3. The factor VIII mutant expression vector according to claim 1, wherein the vector is a vector selected from a group consisting of pCDNA3.1, pGP and pEF.

4. The factor VIII mutant expression vector according to claim 1, which has increased protein expression as compared to an expression vector comprising a polynucleotide encoding factor VIII represented by SEQ ID NO 1, B-domain-deleted factor VIII or single-chain factor VIII.

5. An expression system for expressing a factor VIII mutant, comprising the expression vector according to claim 1.

6. A pharmaceutical composition for preventing or treating hemorrhagic disease or hemorrhage, comprising the expression vector according to claim 1.

7. The pharmaceutical composition for preventing or treating hemorrhagic disease or hemorrhage according to claim 6, wherein the hemorrhagic disease is hemophilia A, hemophilia caused or complicated by an inhibitory antibody against factor VIII or factor Villa, or hemophilia B.

8. The pharmaceutical composition for preventing or treating hemorrhagic disease or hemorrhage according to claim 6, wherein the hemorrhagic disease is one selected from a group consisting of neonatal coagulopathy, severe liver disease, thrombocytopenia, congenital deficiency of factor V, VII, X or XI, and von Willebrand disease with inhibitors against von Willebrand factor.

9. The pharmaceutical composition for preventing or treating hemorrhagic disease or hemorrhage according to claim 6, wherein the hemorrhage is caused by blood loss associated with a high-risk surgical procedure, traumatic blood loss, bone marrow transplantation or cerebral hemorrhage.

10. The pharmaceutical composition for preventing or treating hemorrhagic disease or hemorrhage according to claim 6, which is for gene therapy.
